# EUROPEAN PATENT SPECIFICATION

(11) **EP 2 665 736 B1**
(45) Date of publication and mention of the grant of the patent: **16.09.2015**
(21) Application number: 11709656.0
(22) Date of filing: 21.01.2011
(51) Int. Cl.: C07H 5/00, C07H 13/00, C07H 15/00, A61K 31/704, A61K 47/48, A61K 47/26

(54) **CORONA-LIKE (GUANIDYL)-OLIGOSACCHARIDIC DERIVATIVES AS CELL-PENETRATING ENHANCERS FOR INTRACELLULAR DELIVERY OF COLLOIDAL THERAPEUTIC SYSTEMS**
CORONAARTIGE (GUANIDYL)-OLIGOSACCHARID-DERIVATE ALS ZELLENPENETRIERENDE VERSTÄRKER FÜR DIE INTRAZELLULÄRE VERABREICHUNG VON KOLLOIDALEN THERAPEUTISCHEN SYSTEMEN
DÉRIVÉS (GUANIDYL)-OLIGOSACCHARIDIQUES DE TYPE COURONNE EN TANT QU'AMÉLIORATEURS DE LA PÉNÉTRATION CELLULAIRE POUR L'ADMINISTRATION INTRACELLULAIRE DE SYSTÈMES THÉRAPEUTIQUES COLLOÏDAUX

(43) Date of publication of application: 27.11.2013
(73) Proprietor: Universita' Degli Studi Di Padova, 35122 Padova (IT)
(72) Inventor: CALICETI, Paolo, 35100 Padova (IT); SALMASO, Stefano, 35031 Abano Terme (IT); BERSANI, Sara, 37036 San Martino B.A. (IT)
(74) Representative: Gervasi, Gemma
(86) International application number: PCT/EP2011/050813
(87) International publication number: WO 2012/097876

(56) References cited:
- GOUTAM BISWAS ET AL: "Novel Guanidine-Containing Molecular Transporters Based on Lactose Scaffolds: Lipophilicity Effect on the Intracellular Organellar Selectivity", CHEMISTRY - A EUROPEAN JOURNAL, WILEY - V C H VERLAG GMBH & CO. KGAA, WEINHEIM, DE, vol. 14, 1 January 2008 (2008-01-01), pages 9161-9168, XP007919068, ISSN: 0947-6539, DOI: DOI:10.1002/CHEM.200801160

## Description

### Field of invention

The present invention relates to a class of cell penetrating enhancers having corona-like structures consisting of (guanidyl)-oligosaccharidic derivatives and use thereof as carriers for intracellular delivery of macromolecules and colloidal systems covalently or physically conjugated thereto.

### Background of the invention

The impermeable nature of the biological barriers to large hydrophilic structures is the main reason of poor absorption and inefficient intracellular delivery of proteins, oligonucleotides and colloidal supramolecular systems. Such a poor transmembrane permeability strongly limits the development of convenient non parenteral formulations, transmucosal drug delivery as well as the intracellular delivery of bioactive macromolecules and colloidal drug therapeutic systems, namely polymer conjugates and nanoparticles.

Recently, small regions of proteins termed protein transduction domains (PTDs) or cell penetrating peptides (CPPs) have been demonstrated to possess cell penetrating properties that can be properly exploited to enhance the performance of colloidal therapeutics.

Since about 15 years ago, when penetratin has been recognised as the first cell penetrating enhancer peptide, a variety of CPPs have been developed. They include Tat derived peptides, signal sequence derived peptides and synthetic or chimeric peptides. Most of the recognized CPPs contain cationic, amphiphilic or hydrophobic functions, which promote the cell surface interaction and translocation.

The Tat derived peptides have been demonstrated to be very efficient penetrating agents. They have been designed according to the structure of the HIV-1 Tat, an 86 amino acid nuclear transcription-activating protein constituted by three functional domains. The region responsible for cell uptake and nuclear import contains 6 arginine and 2 lysine residues within a linear sequence of 9 amino acids in random conformation. The presence of short peptide sequences containing basic residues, such as arginines and lysines, is a structural requisite to enable cell-penetrating activity and nucleus translocation of these oligoaminoacids (Green M, Loewenstein PM, 1988; Kalderon D et al., 1984). Accordingly, many homopolymers of arginine have been synthesised and investigated. Studies demonstrated that their internalization efficiency depends on the length of the peptide backbone. Stretches of six (R6) to eight (R8) arginine residues showed the highest cytoplasmic internalization ability (Futaki S et al., 2001). However, the mechanism of the cell translocation is still controversial as either passive or active mechanisms, receptor or absorptive mediated endocytosis have been claimed. Also, the correlation between the structural features of these peptides and the cell uptake behaviour is still under active investigation.

Two molecular intracellular transporters based on lactose scaffolds and seven guanidine residues, which are covalently attached to the oligosaccharide through ester bonds and straight alkyl spacers, are described (Goutam B. et al., 2008). The cellular uptake and intracellular distribution appear to be associated with their lipophilicity.

Although many aspects of CPP activity have not been fully elucidated yet, and a number of contrasting experimental data have been generated, these molecules have a wide relevance in the pharmaceutical and medical fields as they open up interesting perspectives either for enhanced transmucosal absorption, mostly for nasal and intestinal delivery, or for intracellular drug delivery and targeting (Fonseca S et al., 2009). Covalent linkages or simple physical interactions of CPPs have been demonstrated to promote the cell entry of a large variety of hydrophilic compounds ranging from small molecules to plasmid DNA and oligonucleotides (Fisher AA et al., 2009; Veldhoen S et al., 2008), peptides (Bonny C et al., 2001), proteins (Peitz M et al., 2002; Säälik P et al., 2004), as well as nanocarriers such as liposomes, micelles (Torchilin VP, 2008), polymeric particles and quantum-dots (Lewin M et al., 2000; Medintz IL et al., 2008). Nevertheless, many studies highlighted some pitfalls in the use of CPPs in drug delivery such as poor cell uptake, unspecific cell delivery, inconvenient *in vivo* performance, low stability and intrinsic biological activity. Therefore, the combination of these molecules may result ineffective or entail the risk of toxicity (Tréhin R et al. 2004).

### Summary of the invention

Since the structural features of CPPs have been shown paramount to the penetration properties, in order to overcome the drawbacks to the exploitation of CPPs for drug delivery previously mentioned, the first purpose of the present invention is to provide non-peptide molecules with cell penetrating activity. Another purpose is to design molecules displaying higher stability and lower toxicity as compared to CCPs and being devoid of any biological activity. A further purpose is to properly build up unconventional chemical structures suitable for chemical conjugation or physical assembling to macromolecular or supramolecular therapeutic systems for efficient and selective cell targeting.

According to these purposes, the inventors have conceived a novel class of cell penetrating enhancers with unusual corona-like structure suitable to be covalently or physically combined with macromolecular and colloidal systems to promote their cell uptake.

Therefore, in a first aspect an object of present invention are molecules consisting of (guanidyl)-oligosaccharidic derivatives represented by the general formula (I) 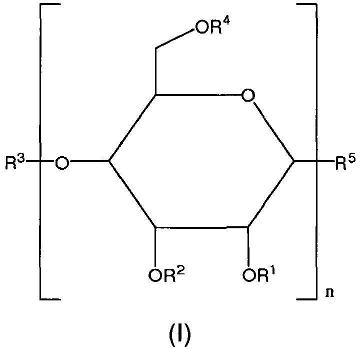 wherein:
R¹, R², R³, R⁴, equal or different from each other are:
   - a hydrogen -H;
   - a linker of formula -CO-R^{I}-X, where R^{I} is a C₂-C₃ alkyl branched chain and where X is a halogen; or
   - a guanidine terminating group of formula -CO-R¹-[CH₂C(CH₃)-CO-NH-R^{II}-NHC(NH₂)=NH]-X, where R^{I} and X have the same meanings previously mentioned for the linker, and R^{II} is a linear or branched C₄-C₈ alkyl chain optionally bearing a -OH or a -COOH group,
   with the proviso that R¹, R², R³, R⁴ represent independently one from the other at least one guanidine terminating group of formula -CO-R^{I}-[CH₂C(CH₃)-CO-NH-R^{II}-NHC(NH₂)=NH]-X;
R⁵ is:
   - a spacer -(CH₃CO)N-R^{III}-Y, where R^{III} is a linear or branched C₈-C₂₂ alkyl chain or an oligo(ethylene glycol) chain derivative of formula -[CH₂CH₂O]ₘ-(CH₂)ₚ where m is an integer selected in the range from 2 to 136 and p is an integer selected in the range from 2 to 6, and Y is a terminating group selected from the carboxyl-, amino-, hydroxyl- or thiol- groups;
n is an integer ranging from 2 to 8,
and salts thereof.

In another aspect a further object of the invention is the use of (guanidyl)-oligosaccharidic derivatives represented by the general formula (I) as carriers for intracellular delivery of macromolecules and colloidal systems physically or covalently conjugated thereto.

Yet, in a further aspect another object of the present invention is the method for preparing said (guanidyl)-oligosaccharidic derivatives represented by the general formula (I) comprising at least the steps of:
- oligosaccharides derivatization on the anomeric carbon of the terminal glycosidic unit with a spacer of formula -HN-R^{III}-Y, where R^{III} and Y have the meanings previously mentioned;
- N-acetylation of the oligoglycosylamino-derivative obtained;
- esterification of oligosaccharide hydroxyl groups with a linker of formula -CO-R^{I}-X, where R^{I} and X have the meanings previously mentioned;
- conjugation of guanidyl residue of formula -[CH₂C(CH₃)-CO-NH-R^{II}-NHC(NH₂)=NH]-X, where R^{II} has the meanings previously mentioned, to the linkers previously introduced on the hydroxyl groups of the oligosaccharidic backbone via atom transfer radical polymerization (ATRP).

Other characteristics and advantages of the present invention will be described in the following detailed description of possible embodiments of corona-like (guanidyl)-oligosaccharidic derivatives of general formula (I).

### Brief description of the figures

**Figure 1** shows the NMR analysis of the corona-like (arginyl)₄-maltosyl-N-acetyl-amino-dodecanoic acid of example 1.
**Figure 2** shows the FT-IR analysis of the corona-like (arginyl)₄-maltosyl-N-acetyl-amino-dodecanoic acid of example 1.
**Figure 3** shows the NMR analysis of the corona-like (arginyl)₄-lactosyl-N-acetyl-amino-dodecanoic acid of example 2.
**Figure 4** shows the FT-IR analysis of the corona-like (arginyl)₄-lactosyl-N-acetyl-amino-dodecanoic acid of example 2.
**Figure 5** shows the NMR analysis of the corona-like (arginyl)₇-maltotriosyl-N-acetyl- amino-dodecanoic acid of example 3.
**Figure 6** shows the FT-IR analysis of the corona-like (arginyl)₇-maltotriosyl-N-acetyl- amino-dodecanoic acid of example 3.
**Figures 7A-B** show the synthesis of a corona-like (arginyl)-maltotriosyl-N-acetyl-amino-dodecanoic acid of example 3, namely the (arginyl)₇-maltotriosyl-N-acetyl-amino-dodecanoic acid: A. 1. maltotriose derivatization with 12-aminododecanoic acid; 2. esterification of the maltotriosyl hydroxyl groups with 2-bromoisobutyryl bromide; B. 3. arginine conjugation via atom transfer radical polymerization (ATRP).
**Figure 8** shows the chemical formula of the corona-like (arginyl)₇-maltotriosyl-N-acetyl-amino-dodecanoic acid of example 3.
**Figures 9A-B** show the synthesis of fluorescent (arginyl)₇-maltotriosyl-N-acetyl-amino-dodecanoyl-BSA.
**Figure 10** shows the relative fluorescence of untreated (black), FITC-BSA treated (white) and (arginyl)₇-maltotriosyl-N-acetyl-amino-dodecanoyl-FITC-BSA treated (grey) lysated MCF-7 cells and MC3T3-E1 cells. Mean values and standard deviations were calculated on the basis of values obtained from five experiments.
**Figure 11** shows the side scatter (SSC-H) versus fluorescence (FL1-H) plots obtained by flow activated cytometry sorting analysis of MCF-7 (A) MC3T3-E1 (B) cells after incubation with plain buffer (A1 and B1), FITC-BSA (A2 and B2) and (arginyl)₇-maltotriosyl-N-acetyl-amino-dodecanoyl-FITC-BSA (A3 and B3).
**Figure 12** shows the confocal microscope images obtained with MCF-7 (A) and MC3T3-E1 (B) incubated for 30 min with FITC-BSA (A1 and B1) and (arginyl)₇-maltotriosyl-N-acetyl-amino-dodecanoyl-FITC-BSA conjugate (A2 and B2) (back = background; dark grey = blue fluorescence; light grey = green fluorescence).
**Figure 13** shows the cytotoxicity profiles obtained by MCF-7 (A) and MC3T3-E1 (B) incubation with increasing concentrations of 2 kDa polyethylenimine (○) and (arginyl)₇-maltotriosyl-N-acetyl-amino-dodecanoic acid (●). Mean values and standard deviations were calculated on the basis of values obtained from six experiments.

### Detailed description of the invention

The corona-like cell penetration enhancers consist in non linear structures different from oligopeptides (i.e. cell penetrating peptides). Their structure is characterized by guanidine group grafting to oligosaccharide cores.

The cell-penetrating enhancers of the invention consisting of corona-like non-linear (guanidyl)-oligosaccharidic derivatives of general formula (I) 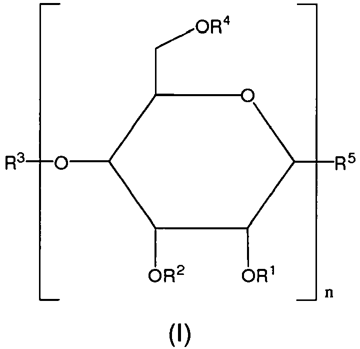 wherein:
R¹, R², R³, R⁴ equal or different from each other are:
   - a hydrogen -H; or
   - a linker of formula -CO-R^{I}-X, where R^{I} is a C₂-C₃ alkyl branched chain and where X is a halogen; or
   - a guanidine terminating group of formula -CO-R^{I}-[CH₂C(CH₃)-CO-NH-R^{II}-NHC(NH₂)=NH]-X, where R^{I} and X have the same meanings previously mentioned for the linker, and R^{II} is a linear or branched C₄-C₈ alkyl chain optionally bearing a -OH or a -COOH group,
   with the proviso that R¹, R², R³, R⁴ represent independently one from the other at least one guanidine terminating group of formula -CO-R^{I}-[CH₂C(CH₃)-CO-NH-R^{II}-NHC(NH₂)=NH]-X;
R⁵ is:
   - a spacer -(CH₃CO)N-R^{III}-Y, where R^{III} is a linear or branched C₈-C₂₂ alkyl chain or a linear oligo(ethylene glycol) chain derivative of formula - [CH₂CH₂O]ₘ-(CH₂)ₚ where m is an integer selected in the range from 2 to 136 and p is an integer selected in the range from 2 to 6, and Y is a terminating group selected from the carboxyl-, amino-, hydroxyl- or thiol- groups;
n is an integer selected in the range from 2 to 8,
have been designed keeping in mind the oligo-arginyl structure of the Tat protein (SwissProt P04608; GenBank K03455) fragment 49-57, which is involved in the trans-membrane translocation. Said corona-like non-linear (guanidyl)-oligosaccharidic derivatives of general formula (I) are specifically conceived for conjugation to large size systems, namely proteins, oligonucleotides or colloidal drug carriers in order to promote their cell entry. The synthesis of the new cell penetrating enhancer was set up in order to obtain a non-conventional architecture by introducing guanidyl moieties into a core anchoring structure. The oligosaccharides were selected as anchoring molecules, since their multifunctional structure can be properly derivatised through simple chemical protocols with guanidyl residues and a spacer arm to produce a non peptide "corona-like" construct for conjugation to macromolecules or supramolecular drug delivery systems.

In fact, the (guanidyl)-oligosaccharidic derivatives of formula (I) can easily conjugate bioactive macromolecules, both specific macromolecular or supramolecular colloidal structures, by means of a covalent binding through the spacer represented by R⁵ bearing the latter a terminating functional group, whilst the cationic features of these (guanidyl)-oligosaccharidic derivatives can be exploited for a physical combination with said macromolecules or colloidal systems. Said macromolecules or supramolecular colloidal structures can be peptides, proteins, oligonucleotides, siRNA, genes, polymer therapeutics, namely dendrimers and other bioconjugates, or supramolecular cargoes, namely liposomes, micelles, nanoparticles.

For the purposes of the invention, the oligosaccharide core is at least formed by two and up to 8 (and preferably 2 or 3) glycosidic units and said oligosaccharides are preferably selected from the group consisting of lactose, mannose, cellobiose, maltose, isomaltose, maltotriose, maltotetraose, maltopentose, maltohexaose, maltohepatose, maltooctaose. The preferred oligosaccharides are lactose, maltose, maltotriose, maltoheptaose and the most preferred oligosaccharides are lactose, maltose and maltotriose.

Moreover, for the corona-like (guanidyl)-oligosaccharidic derivatives of formula (I) in R¹, R², R³, R⁴ R^{I} is preferably an isopropyl group and X is preferably Cl or Br, and when in R¹, R², R³, R⁴ are guanidyl residue of formula -[CH₂C(CH₃)-CO-NH-R^{II}-NHC(NH₂)=NH]-X R^{II} is a C₄ alkyl chain bearing a -COOH group. As for the preferred meanings of R⁵, R^{III} is preferably a C₁₀-C₁₈ alkyl chain and Y is a terminating functional group preferably selected from -COOH or -NH₂. When R^{III} is a linear oligo(ethylene glycol) chain -[CH₂CH₂O]ₘ- its preferred molecular weight is from 200 to 3400 Da and m is selected from 5 to 77. The most preferred linker of formula - CO-R^{I}-X is 2-bromoisobutyryl group and the most preferred spacer R⁵ is N-acetyl-12-aminododecanoyl acid.

In preferred embodiments in the (guanidyl)-oligosaccharidic derivatives of general formula (I) R¹, R², R³, R⁴, independently one from the other, are at least 2 and preferably from 4, when the oligosaccharidic core is a disaccharide, to 20, when the oligosaccharidic core is a heptasaccharide, guanidine terminating group of formula -CO-R^{I}-[CH₂C(CH₃)-CO-NH-R^{II}-NHC(NH₂)=NH]-X. In most preferred embodiments R¹, R², R³, R⁴ are all equal to guanidine terminating groups of formula -CO-R^{I}-[CH₂C(CH₃)-CO-NH-R^{II}-NHC(NH₂)=NH]-X. The preferred guanidine terminating groups of formula -CO-R^{I}-[CH₂C(CH₃)-CO-NH-R^{II}-NHC(NH₂)=NH]-X are selected from 4 to 7, when the oligosaccharidic core is a disaccharide, and from 4 to 20, when the oligosaccharidic core is a heptasaccharide, where R^{I}, X and R^{II} have the preferred meanings above indicated.

The corona-like (guanidyl)-oligosaccharidic derivative of the invention was synthesised according to the multi-step chemical protocol involving as herein previously indicated:
- oligosaccharides derivatization on the anomeric carbon of the terminal glycosidic unit with a spacer of formula -NH-R^{III}-Y selected from -NH-(linear or branched C₈-C₂₂ alkyl chain)-COOH, -NH-(linear or branched C₈-C₂₂ alkyl chain)-OH, -NH-(linear or branched C₈-C₂₂ alkyl chain)-SH, -NH-(linear or branched C₈-C₂₂ alkyl chain)-NH₂,-NH-(CH₂CH₂O)ₘ(CH₂)ₚ-COOH, -NH-(CH₂CH₂O)ₘ(CH₂)ₚ-OH, -NH-(CH₂CH₂O)ₘ(CH₂)ₚ-SH, -NH-(CH₂CH₂O)ₘ(CH₂)ₚ-NH [m=2-136; p=2-6],
- N-acetylation of the oligoglycosylimino-group function obtained;
- esterification of the remaining oligosaccharide hydroxyl groups with the linker of formula X-CO-R^{I}-X, where R^{I} and X have the meanings previously mentioned, and preferably with 2-bromoisobutyryl bromide;
- guanidyl conjugation to the arms introduced on the hydroxyl groups of the oligosaccharidic backbone with the linkers via atom transfer radical polymerization (ATRP).

The first step of derivatization of a selected oligosaccharide is obtained by reaction of the amino group of the selected spacer R⁵, preferably 12-amino dodecanoic acid, with the anomeric carbon of selected oligosaccharide (preferably maltose or maltotriose). The reaction is carried out in methanol/acetic acid (95/5 by volume) at 50-65 °C. The resulting amino group is stabilised by amino acetylation using acetic anhydride to avoid the detachment of the amino-spacer residue. The acetylation is carried out for up to 96 hours at room temperature. The oligosaccharide derivative obtained is purified by precipitation in ethyl ether.

In the second step said N-acetylated glycosyl-amino-spacer derivative is esterificated at the saccharides OH functions with a selected linker (preferably 2-bromo-isobutyryl-bromide) in order to obtain a multi-armed oligosaccharide initiator for ATRP polymerization. The linker conjugation is carried out in chloroform at 0°C in the presence of triethylamine. The conjugation of guanidyl residues to the multi-armed oligosaccharide initiator is carried out in dimethylsulfoxide by ATRP polymerization using methacryloyl-guanidyl derivative (preferentially methacryloyl-arginine) as monomer and tris[(2-pyridyl)methyl]amine (TPMA) as ligand for Cu(I). Both methacryloyl-arginyl derivative and TPMA are synthesised according to modified protocols reported in the literature known to an artisan skilled in the art. The ATRP reaction is carried out under nitrogen atmosphere to avoid the catalyst inactivation by oxygen oxidation of Cu(I) to Cu(II).

The evaluation assays for cell-penetrating activity and cytotoxicity were performed on the lead derivative (arginyl)₇-maltotriosyl-N-acetyl-amino-dodecanoic acid.

The cell penetrating properties of the new (arginyl)₇-maltotriosyl-N-acetyl-amino-dodecanoyl derivative were evaluated using BSA as cargo molecule because this hydrophilic macromolecule is not taken up by cells. The human MCF-7 breast adenocarcinoma and murine MC3T3-E1 embryonic fibroblast cell lines were chosen because they do not have phagocytic properties.

The cell culture analysis by fluorescence spectrometry, fluorescence activated cell sorting and confocal microscopy showed that the corona-like (arginyl)₇-maltotriosyl-N-acetyl-amino-dodecanoyl derivative possesses excellent cell penetration properties.

The quantitative fluorescence analysis showed that, although both cell lines undergo extensive (arginyl)₇-maltotriosyl-N-acetyl-amino-dodecanoyl-FITC-BSA uptake, the protein internalization obtained with MC3T3-E1 was slightly higher than that obtained with MCF-7. On the other hand, the cytofluorimetric data showed that higher fraction of MC3T3-E1 cells were involved in the (arginyl)₇-maltotriosyl-N-acetyl-amino-dodecanoyl-FITC-BSA uptake with respect to the MCF-7 cells. These results seem to suggest that the novel cell penetration enhancer display cell dependent translocation properties possibly exploiting different penetration mechanisms.

The confocal microscopy confirmed the remarkable cell uptake of (arginyl)₇-maltotriosyl-N-acetyl-amino-dodecanoyl-FITC-BSA. Furthermore, the confocal images showed a punctuated fluorescence distribution, which corroborates the hypothesis that the macromolecular cargo is taken up by endocytosis pathway rather than by simple passive diffusion. Also, the lack of fluorescence in the nucleus seems to indicate that the transmembrane translocation is limited to the cytoplasm compartment. Finally, the novel non-linear (arginyl)₇-oligosaccharide derivative was found to possess negligible toxicity. The IC50 was in fact higher that 100 µM, which corresponds to a very high concentration.

This is a relevant issue in favour of these new cell-penetrating products, which undergoes intracellular localisation. Furthermore, low toxicity is required because these products can be used also in high doses to enhance the drug absorption of non-invasive formulations. In any case, absence or low toxicity is a requisite for its exploitation in drug delivery.

Therefore, the corona-like (guanidyl)-oligosaccharidic derivatives provided by the present invention fully fulfil the purposes of the present invention and can be usefully employed as carriers for intracellular delivery of macromolecules or colloidal nanoparticles through a covalent binding or a physical combination with the same.

The invention is further illustrated by the following examples.

### EXAMPLES

### Materials

Maltose monohydrate [4-O-α-D-glucopyranosyl-D-glucose], Lactose monohydrate [4-O-β-D-galactopyranosyl-α,β-D-glucose], Maltotriose [*O*-α-D-glucopyranosyl-(1,4)-*O*-α-D-glucopyranosyl-(1,4)-D-glucose], Maltoheptaose {O-α-D-glucopyranosyl-[(1-4)-O-α-D-glucopyranosyl]₅(1-4)-D-glucose}, 12-aminododecanoic acid, acetic anhydride, 2-bromo-isobutyryl bromide, triethylamine, ascorbic acid, 1-ethyl-3-(3-dimethylaminopropyl) carbodiimide hydrochloride (EDC), polyethylenimine (PEI, 2 kDa), fetal bovine serum (FBS), RPMI-1640 medium, Dulbecco's modified essential medium (D-MEM), penicillin (10000 UI/mL) -streptomycin (10 mg/mL) - amphotericin B (25 µg/mL) stabilised solution, L-glutamine (200 mM), Dulbecco's phosphate buffered saline (D-PBS), trypsin-EDTA solution (2.5 %w/v), 3-(4,5-dimethylthiazol-2-yl)-2,5-diphenyltetrazolium bromide (MTT) were purchased from Sigma-Aldrich (St. Louis, MO, USA).

Amino/carboxyl terminating heterobifunctional poly(eththlene glycol) (NH₂-PEG-COOH, MW 3.4 kDa) was obtained by Nektar (Huntsville, AL, USA).

L-Arginine hydrochloride was furnished by SERVA Electrophoresis (Heidelberg, Germany).

Fluorescein isothiocyanate (FITC), 2-picolyl chloride hydrochloride, 2-picolylamine, copper(I) bromide (CuBr), bovine serum albumin - fraction V (BSA), Triton X-100, paraformaldehyde were purchased from Fluka (Buchs SG, Switzerland). Vectashield^{®} Mounting Medium with DAPI was from Vector Laboratories (Peterborough, UK).

All other reagents were obtained from Fluka Analytical or Sigma-Aldrich.

The solvents of analytical or HPLC grade were furnished by Carlo Erba (Milan, Italy), VWR International (Lutterworth, UK) and Sigma-Aldrich (St. Louis, MO, USA).

Cell culture plates and flask, 96-well flat bottomed microassay plate and four-well chambered slides were purchased from BD Falcon (BD Bioscience, NJ, USA).

As example of embodiments of the invention novel corona-like (guanidyl)-oligosaccharidic derivatives were synthesised according to the multi-step chemical protocol previously described in detail. The intermediate and final products were characterised by ¹H-NMR, FT-IR, mass spectrometry, colorimetric assays and elemental analysis.

In the examples 1 and 4 are given in full details the preparations of the (arginyl)₄-maltosyl-N-acetyl-amino-dodecanoic acid, (arginyl)₄-lactosyl-N-acetyl-amino-dodecanoic acid, (arginyl)₇-maltotriosyl-N-acetyl-amino-dodecanoic acid and oligo(arginyl)-maltoheptaosyl-N-acetyl-amino-poly(ethylene glycol)-carboxyl acid.

### Example 1. Synthesis of the corona-like (arginyl)₄-maltosyl-N-acetyl-amino-dodecanoic acid

### Synthesis of maltosyl-N-acetyl-amino-dodecanoic acid

One gram of maltose monohydrate (2.775 mmoles) was dissolved in 20 mL of CH₃OH/1.5% acetic acid containing 1.195 g of 12-aminododecanoic acid (5.55 mmoles). The suspension was thermostated at 65°C and magnetically stirred. After 24 hours, the reaction mixture was cooled to 0°C and added of 2.5 mL of acetic anhydride (26.47 mmoles). The solution was maintained under stirring for 24 hours at 40°C and then cooled to room temperature and left under stirring. The precipitate was removed by centrifugation and the solution was reduced to 4 mL under vacuum and dropped into 200 mL of diethyl ether. The precipitate was collected by filtration and desiccated under reduced pressure. The product yield was 1.6 g.

The purified material was analyzed by mass spectrometry, ¹H-NMR, and RP-HPLC using a Luna C18 column (250x4.6mm, Phenomenex, Torrance, CA, USA) eluted with a gradient of H₂O/0.05% trifluoroacetic acid (A) and acetonitrile/0.05% trifluoroacetic acid (B) (0-3 min 15% B, 3-27 min from 15% to 90% B), the UV detector was set at 220 nm. ESI-MS *[m*/*z]:* 580.28 (M-H⁺)¹⁻ [calcd for C₂₆H₄₇NO₁₃: 581.30].
¹H-NMR (D₂O): *δ* ppm 5.22 d [1 H, H anomeric], ppm 3.97-3.60 *broad* m [14H, H maltose], ppm 3.43 t [2H, J = 9.3 Hz, -NC**H₂**-], ppm 2.30 t [2H, J = 7.3 Hz, - C**H₂**COOH), ppm 2.16 s [3H, -NCOC**H₃**], ppm 1.58 *broad* s [4H, - NCH₂C**H₂**(CH₂)₇C**H₂**CH₂COOH], ppm 1.30 s [14H, -N(CH₂)₂(C**H₂**)₇(CH₂)₂COOH].
RP-HPLC (C18): 14.6 min retention time.

### Synthesis of (2-bromoisobutyryl)₄-maltosyl-N-acetyl-amino-dodecanoic acid

The synthesis of (2-bromoisobutyryl)₄-maltosyl-N-acetyl-amino-dodecanoic acid was carried out according to a modified procedure reported in the literature (Stenzel-Rosenbaum MH et al., 2001). Maltosyl-N-acetyl-amino-dodecanoic acid previously prepared (200 mg, 0.34 mmoles) was suspended in 5 mL of anhydrous dichloromethane containing 380 µl of triethylamine (2.75 mmoles). The solution was cooled in an ice bath and added of 408 µl of 2-bromo-isobutyryl-bromide (3.30 mmoles) in 15 min, and left under stirring at room temperature. After 60 hours the mixture was filtered and the organic solution treated three times with 15 mL iced water, three times with 10 mL of 0.1 N NaOH and finally three times with 15 mL of iced water. The organic phase was dried with anhydrous Na₂SO₄, filtered and concentrated under vacuum. The resulting red-brown solid was dissolved in 2 mL acetone and added of 250 mL cold water under vigorous stirring. Acetone was removed under vacuum and the aqueous suspension was lyophilised. The yield of the product was 285 mg.

The purified material was analysed by elemental analysis and ¹H-NMR.

Elemental analysis: found C, 42.4%; H, 5.6%; N, 1.2%; Br, 25.8% (O, 25.7%) [calcd for (2-bromoisobutyryl)₄-N-acetyl-maltosyl-amino-dodecanoic acid (C₄₂H₆₇Br₄NO₁₇): C, 42.8%; H, 5.7%; N, 1.2%; Br, 27.1% (O, 23.2%)].
¹H NMR (CDCl₃): *δ* ppm 4.60-3.30 (bm, 12H of glycosyl moiety), 3.29 (bm, 2H, λ-CH₂-), 2.15 (m, 2H, α-CH₂-), 1.96 (bs, 24H, CH₃), 1.94 (s, 3H, acetyl-CH₃), 1.63 (bm, 4H, i-CH₂- and β-CH₂-), 1.33-1.22 (bm, 14H, -CH₂- of alkyl chain).

### Synthesis of methacryloyl-arginine

Methacryloyl-arginine was synthesised according to the procedure reported in literature [US 6,703,468]. Briefly, 2.1 g of arginine hydrochloride (10 mmoles) dissolved in 20 mL of saturated NaHCO₃ water solution was cooled to 0°C and added dropwise of 970 µl of methacryloyl chloride (10 mmoles) under vigorous stirring. After 1 hour the pH of mixture was adjusted to 1 with hydrochloric acid and the solution was saturated with sodium chloride, filtered, washed three times with 30 mL of ethyl acetate, and finally extracted three times with 20 mL of 1:1 of ethyl acetate/isopropanol mixture. The ethyl acetate/isopropanol volumes were pooled and concentrated under reduced pressure. The resulting suspension was filtered to remove the precipitate and the solution was desiccated. The colourless syrup was dissolved in 50 mL water and freeze-dried. The final product yield was 2.4 g.

The product was analysed by ¹H NMR, FT-IR and ESI-MS.
¹H-NMR (D₂O): *δ* ppm 5.69 (s, 1 H, H₂C=C), 5.47 (s, 1 H, H₂C=C), 4.41 (dd, *J =* 9.18, 5.09 Hz, 1 H, α-CH), 3.20 (t, *J* = 6.77, 2H, δ-CH₂), 1.91 (s, 3H, CH₃-), 1.85-1.72 (m, 2H, γ-CH₂), 1.72-1.58 (m, 2H, β-CH₂).
FT-IR (KBr): *v* (cm⁻¹) 3368 (OH), 3150 (NHC(NH₂)₂⁺), 1654 (C=C, C=O and C=N), 1528 (CNH₂ or CNH₃⁺).
ESI-MS *[m*/*z]:* 243.13 (M+H⁺)¹⁺ [calcd for C₁₀H₁₈N₄O₃: 242.14].

The purity of the compound was assessed by the integration of the ¹H-NMR triplet at 3.20 ppm compared with methacryloyl singlets at 5.69 and 5.47 ppm (99 %).

### Synthesis of tris[(2-pyridyl)methyl]amine (TPMA)

Tris[(2-pyridyl)methyl]amine (TPMA) was prepared according to a modified protocol described by Tyeklàr et al. (Tyeklàr Z et al. 1993). Ten grams of 2-pycolil chloride (61.0 mmoles) were dissolved in 25 mL of distilled water and the mixture was cooled to 0°C and added of 12 mL of 5 N NaOH. The solution was added of 3.2 mL of 2-(aminomethyl)pyridine (30.5 mmoles) and 50 mL of dichloromethane. The reaction mixture was allowed to reach room temperature under vigorous stirring and the pH 9.5 was maintained by 5.0 N NaOH addition. After 48 hours the organic phase was washed three times with 20 mL of 2.5 N NaOH and then dried with anhydrous Na₂SO₄ and filtered. The organic solvent was eliminated under vacuum. The TPMA was recovered by treating the resulting waxy red-brown product three times with 25 mL of boiling petroleum ether. The organic phase was then desiccated under vacuum. The final product yield was 5.6 g.

The product was analysed by ¹H-NMR and ESI-MS.
¹H-NMR (CDCl₃): *δ* ppm 8.53 (d, *J* = 4.86, 3H, Py-H₆), 7.65 (t, *J* = 7.71, 3H, Py-H₄), 7.58 (d, *J* = 7.71, 3H, Py-H₃), 7.14 (t, *J* = 6.06, 3H, Py-H₅), 3.88 (s, 6H, -CH₂-). ESI-MS [*m*/*z*]: 291.15 (M+H⁺)¹⁺ and 313.14 (M+Na⁺)¹⁺ [calcd for C₁₈H₁₈N₄: 290.15].

### Synthesis of arginyl₄-maltosyl-N-acetyl-amino-dodecanoic acid

TPMA (45.5 mg, 0.16 mmoles), CuBr (22.49 mg, 0.16 mmoles) and ascorbic acid (0.34 mg, 1.96x10⁻³ mmoles) were dissolved in 2.0 mL of nitrogen purged DMSO and added to 0.5 mL of 0.32 g/mL methacryloyl-arginine previously prepared (0.66 mmoles) in DMSO under stirring at 55°C. The solution was added of 0.5 mL of 92.7 mg/mL of (2-bromoisobutyryl)₄-maltosyl-N-acetyl-amino-dodecanoic acid (0.04 mmoles) in DMSO. The reaction mixture was maintained under nitrogen atmosphere for 12 hours and then was exposed to the air. After 30 min the solution was added dropwise to 200 mL of cold 1:1 acetone/diethyl ether mixture containing 1% acetic acid. The precipitate was desiccated under vacuum, redissolved in 5 mL of warm methanol and re-precipitated by dropping into 200 mL of the acetone/diethyl ether/acetic acid mixture. The product yield was 47.8% corresponding to 40.2 mg.

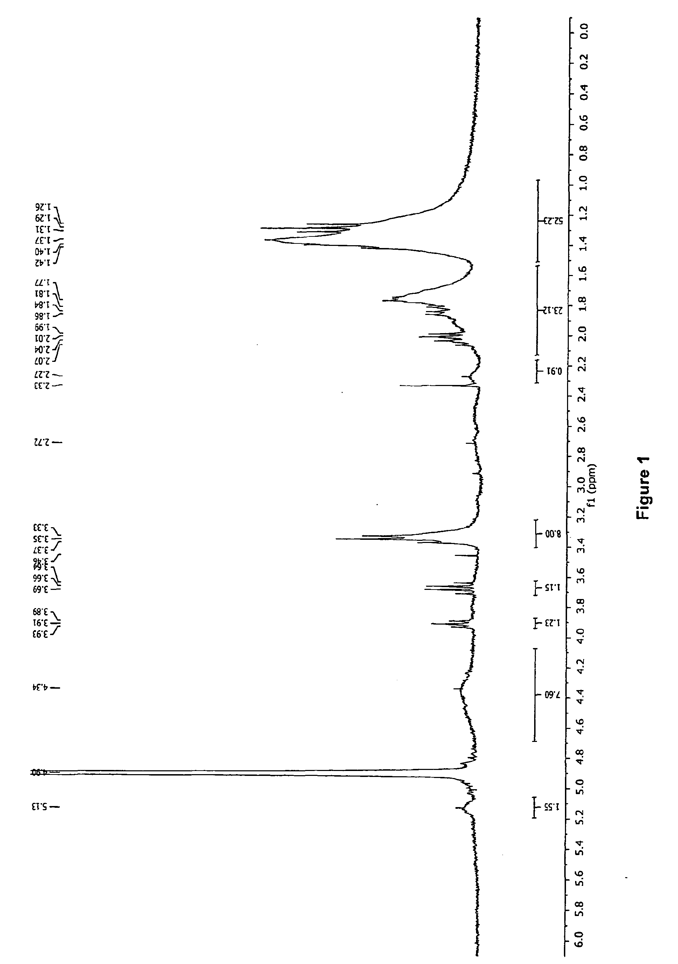

The arginine content in the final product was evaluated by Sakaguchi assay (Sakaguchi S, 1925). The experimental data were referred to a calibration curve obtained with standard solutions of 0-0.6 µM guanidinium content (y = 7.3955x-0.0386, R²=0.99). The final product was also analysed by elemental analysis, ¹H-NMR (FIG. 1 ).

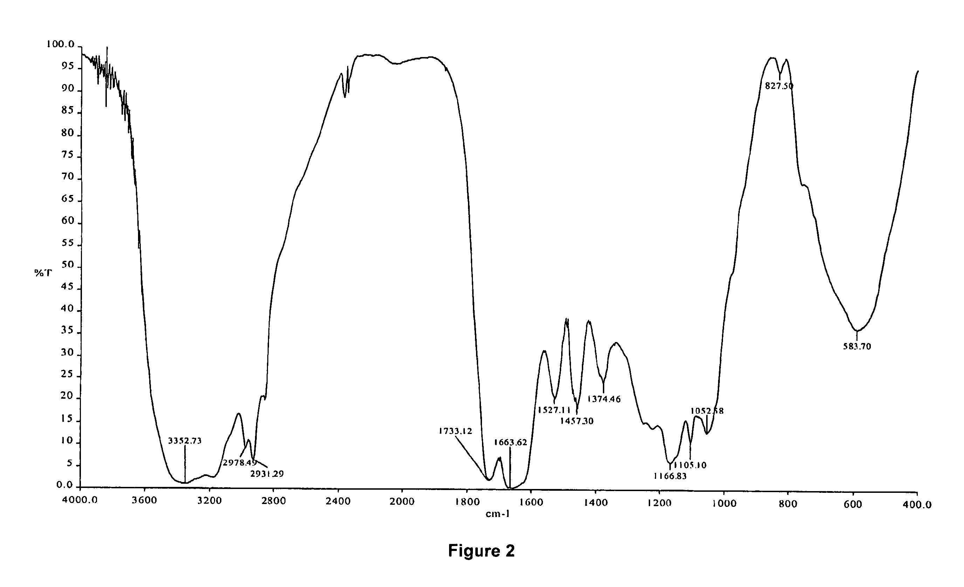

Elemental analysis: found C, 44.9%; H, 6.9%; N, 10.3%; Br, 21.3% (O, 16.6%); [calcd for (arginyl)₄-maltosyl-N-acetyl-amino-dodecanoic acid (C₈₂H₁₃₉Br₄N₁₇O₂₉): C, 45.9%; H, 6.5%; N, 11.1%; Br, 14.9% (O, 21.6%) and calcd for (arginyl)₄-maltosyl-N-acetyl-amino-dodecanoic acid HBr salt (C₈₂H₁₃₉Br₄N₁₇O₂₉·4HBr): C, 39.9%; H, 5.8%; N, 9.6%; Br, 25.9% (O, 18.8%)].
FT-IR: *v* (cm⁻¹) 3353 and 3176 [NHC(NH₂)₂⁺], 2931 (CH), 1733 (C=O), 1664 (C=N). (FIG. 2)

### Example 2. Synthesis of the corona-like (arginyl)₄-lactosyl-N-acetyl-amino-dodecanoic acid

### Synthesis of lactosyl-N-acetyl-amino- dodecanoic acid

Two hundred and fifty milligrams of lactose monohydrate (0.694 mmoles) were dissolved in 10 mL of CH₃OH/1.5% acetic acid containing 299 mg of 12-aminododecanoic acid (1.39 mmoles). The suspension was thermostated at 65°C and magnetically stirred. After 24 hours, the reaction mixture was cooled to 0°C and added of 0.62 mL of acetic anhydride (6.59 mmoles). The solution was maintained under stirring for 24 hours at 40°C and then cooled to room temperature and left under stirring. The precipitate was removed by centrifugation and the solution was reduced to 4 mL under vacuum and dropped into 200 mL of diethyl ether. The precipitate was collected by filtration and desiccated under reduced pressure. The product yield was 294 mg.

The purified material was analyzed by mass spectrometry, elemental analysis, ¹H-NMR and RP-HPLC using a Luna C18 column (250×4.6mm, Phenomenex, Torrance, CA, USA) eluted with a gradient of H₂O/0.05% trifluoroacetic acid (A) and acetonitrile/0.05% trifluoroacetic acid (B) (0-3 min 15% B, 3-27 min from 15% to 90% B), the UV detector was set at 220 nm.
ESI-MS *[m*/*z]:* 580.28 (M-H⁺)¹⁻ [calcd for C₂₆H₄₇NO₁₃: 581.30].
Elemental analysis: found C, 53.6%; H, 8.3%; N, 2.7% (O, 35.4%); [calcd for lactosyl-N-acetyl-amino-dodecanoic acid (C₂₆H₄₇NO₁₃): C, 53.7%; H, 8.1%; N, 2.4% (O, 35.8%)].
¹H-NMR (D₂O): *δ* ppm 4.55 d [1 H, H anomeric], ppm 3.97-3.60 *broad* m [14H, H lactose], ppm 3.50 t [2H, -NC**H₂**-], ppm 2.35 t [2H, -C**H₂**COOH), ppm 2.18 s [3H, - NCOC**H₃**], ppm 1.60 *broad* s [4H, -NCH₂C**H₂**(CH₂)₇C**H₂**CH₂COOH], ppm 1.30 bs [14H, -N(CH₂)₂(C**H₂**)₇(CH₂)₂COOH].
RP-HPLC (C18): 16.8 min retention time.

### Synthesis of (2-bromoisobutyryl)₄-lactosyl-N-acetyl-amino-dodecanoic acid

The synthesis of (2-bromoisobutyryl)₄-lactosyl-N-acetyl-amino-dodecanoic acid was carried out according to a modified procedure reported in the literature [*ref. cit*.]. Lactosyl-N-acetyl-amino-dodecanoic acid previously prepared (200 mg, 0.34 mmoles) was suspended in 5 mL of anhydrous dichloromethane containing 380 µl of triethylamine (2.75 mmoles). The solution was cooled in an ice bath and added of 408 µl of 2-bromo-isobutyryl-bromide (3.30 mmoles) in 15 min, and left under stirring at room temperature. After 60 hours the mixture was filtered and the organic solution treated three times with 15 mL iced water, three times with 10 mL of 0.1 N NaOH and finally three times with 15 mL of iced water. The organic phase was dried with anhydrous Na₂SO₄, filtered and concentrated under vacuum. The resulting red-brown solid was dissolved in 2 mL acetone and added of 250 mL cold water under vigorous stirring. Acetone was removed under vacuum and the aqueous suspension was lyophilised. The yield of the product was 359 mg.

The purified material was analysed by elemental analysis and ¹H-NMR. Elemental analysis: found C, 41.3%; H, 5.9%; N,1.4%; Br, 26.4% (O, 25.0%) [calcd for (2-bromoisobutyryl)₄-N-acetyl-maltosylamino-dodecanoic acid (C₄₂H₆₇Br₄NO₁₇): C, 42.8%; H, 5.7%; N, 1.2%; Br, 27.1% (O, 23.2%)].
¹H NMR (CDCl₃): *δ* ppm 4.60-3.30 (bm, 12H of glycosyl moiety), 3.29 (bm, 2H, λ-CH₂-), 2.15 (m, 2H, α-CH₂-), 1.96 (bs, 24H, CH₃), 1.94 (s, 3H, acetyl-CH₃), 1.63 (bm, 4H, ℩-CH₂- and β-CH₂-), 1.33-1.22 (bm, 14H, -CH₂- of alkyl chain.

### Synthesis of methacryloyl-arginine

Methacryloyl-arginine was synthesised as described in example 1.

### Synthesis of tris[(2-pyridyl)methyl]amine (TPMA)

Tris[(2-pyridyl)methyl]amine (TPMA) was prepared as described in example 1.

### Synthesis of (arginyl)₄-lactosyl-N-acetyl-amino-dodecanoic acid

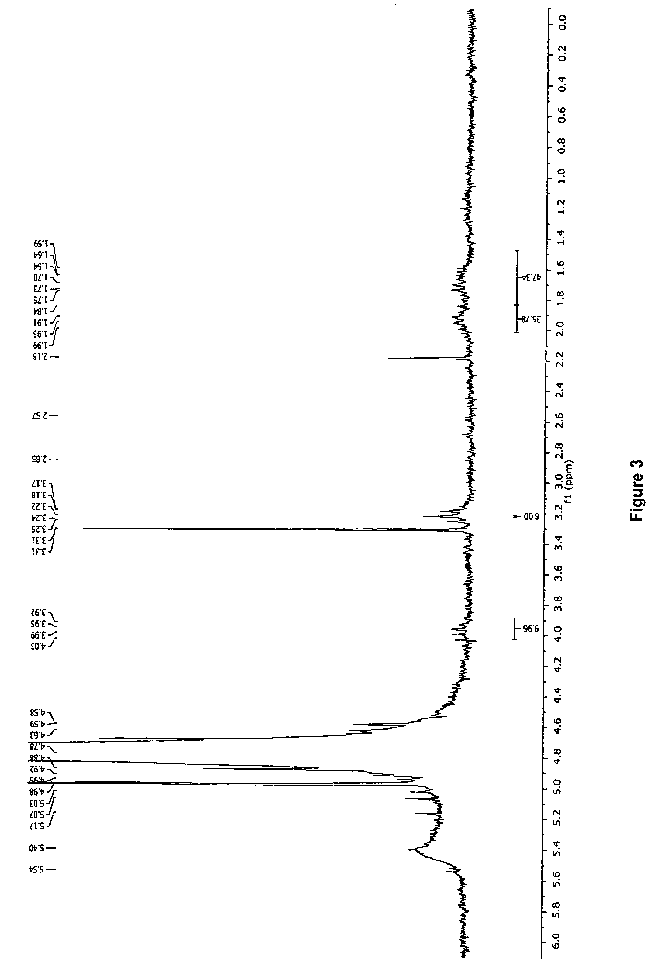

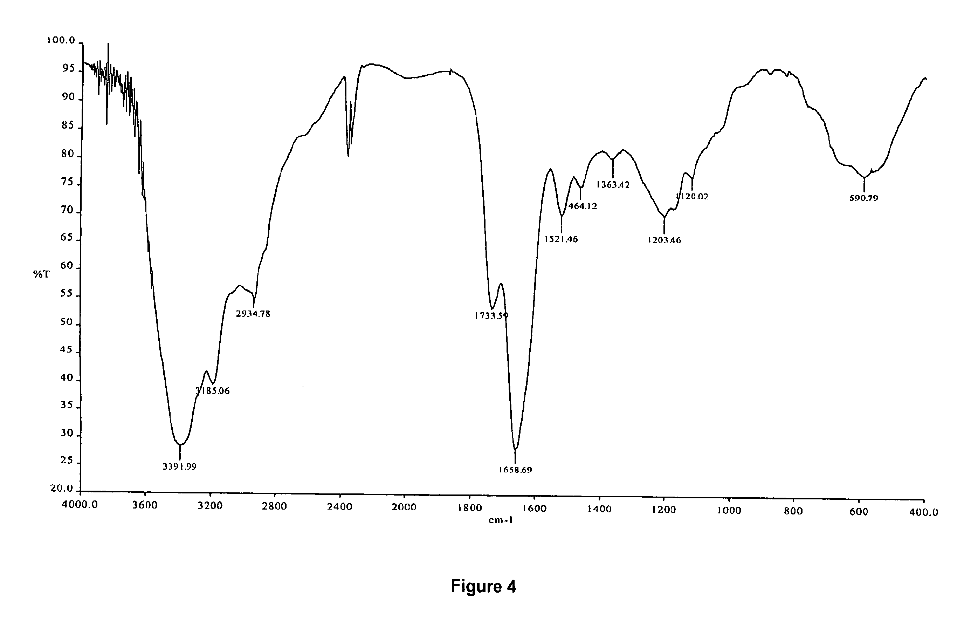

TPMA (49.3 mg, 0.17 mmoles), CuBr (24.4 mg, 0.17 mmoles) and ascorbic acid (0.37 mg, 2.12x10⁻³ mmoles) were dissolved in 2.0 mL of nitrogen purged DMSO and added to 0.5 mL of 0.34 g/mL methacryloyl-arginine previously prepared (0.7 mmoles) in DMSO under stirring at 55°C. The solution was added of 0.5 mL of 98.6 mg/mL of (2-bromoisobutyryl)₄-lactosyl-N-acetyl-amino-dodecanoic acid (0.042 mmoles) in DMSO. The reaction mixture was maintained under nitrogen atmosphere for 12 hours and then was exposed to the air. After 30 min the solution was added dropwise to 200 mL of cold 1:1 acetone/diethyl ether mixture containing 1% acetic acid. The precipitate was desiccated under vacuum, redissolved in 5 mL of warm methanol and re-precipitated by dropping into 200 mL of the acetone/diethyl ether/acetic acid mixture. The product yield was 25.3 mg. The arginine content in the final product was evaluated by Sakaguchi assay (Sakaguchi S, 1925). The experimental data were referred to a calibration curve obtained with standard solutions of 0-0.6 µM guanidinium content (y = 7.3955x-0.0386, R²=0.99). The final product was also analysed by elemental analysis, ¹H-NMR (FIG. 3).
Elemental analysis: found C, 45.3%; H, 6.3%; N, 10.9%; Br, 15.8% (O, 21.7%); [calcd for (arginyl)₄-lactosyl-N-acetyl-amino-dodecanoic acid (C₈₂H₁₃₉Br₄N₁₇O₂₉): C, 45.9%; H, 6.5%; N, 11.1%; Br, 14.9% (O, 21.6%) and calcd for (arginyl)₄-lactosyl-N-acetyl-amino-dodecanoic acid HBr salt (C₈₂H₁₃₉Br₄N₁₇O₂₉·4HBr): C, 39.9%; H, 5.8%; N, 9.6%; Br, 25.9% (O, 18.8%)].
FT-IR: *v* (cm⁻¹) 3392 and 3185 [NHC(NH₂)₂⁺], 2935 (CH), 1734 (C=O), 1659 (C=N). (FIG. 4)

### Example 3. Synthesis of the corona-like (arginyl)₇-maltotriosyl-N-acetyl- amino-dodecanoic acid

### Synthesis of maltotriosyl-N-acetyl-amino-dodecanoic acid

One gram of 12-aminododecanoic acid (4.6 mmoles) was dissolved in 15 mL of CH₃OH/5% acetic acid. The solution was thermostated at 60 °C and added of 1.2 g maltotriose (2.4 mmoles). After 24 hours, 7.5 mL of acetic anhydride (79.4 mmoles) were added to the mixture and the solution was maintained under stirring for 96 hours at room temperature. The volume was reduced to 4 mL under vacuum and added of 3 mL of 30% ammonia solution. After 12 hours, the solution was desiccated under vacuum, and the dried product was redissolved in 3 mL of methanol and added dropwise to 200 mL of diethyl ether. The precipitate was collected by filtration and desiccated under vacuum. The reaction yield was 1.2 g. The final product was analyzed by RP-HPLC using a Luna C18 column (250x4.6mm, Phenomenex, Torrance, CA, USA) eluted with a gradient of H₂O/0.05% trifluoroacetic acid (A) and acetonitrile/0.05% trifluoroacetic acid (B) (0-3 min 10% B, 3-27 min from 10% to 80% B). The UV detector was set at 220 nm. The product was also characterized by elemental analysis, mass spectrometry (ESI-MS; ESI-TOF Applied Biosystems Mariner Foster City, CA, USA), ¹H-NMR (AMX 300 MHz, Bruker Spectrospin, Fallanden, Switzerland) and FT-IR (FT-IR 1600, Perkin-Elmer, Norworlk, CT, USA).
RP-HPLC (C18): 16.3 min retention time.
Elemental analysis: C, 51.2%, H, 7.9%, N, 1.7% (O, 39.2%) [calcd for C₃₂H₅₇NO₁₈: C, 51.7%, H, 7.7%, N, 1.8% (O, 39.8%)].
ESI-MS *[m*/*z]:* 742.34 (M-H⁺)¹⁻ [calcd for C₃₂H₅₇NO₁₈: 743.36].
¹H-NMR (D₂O): *δ* ppm 5.40 (d, *J* = 3.26, 2H, anomeric), 5.23 (d, *J* = 3.79, 1 H, anomeric), 4.20-3.60 (bm, 18H of glycosyl moiety), 3.55 (t, *J* = 9.3, 2H, λ-CH₂-), 2.33 (t, *J =* 7.2, 2H, α-CH₂-), 2.18 (bs, 3H, acetyl-CH₃), 1.59 (bm, 4H, ℩-CH₂- and β-CH₂-), 1.29 (bs, 14H, -CH₂- of alkyl chain). NMR signals were indicated as singlet (s), broad singlet (bs), doublet (d), triplet (t), multiplet (m) and broad multiplet (bm); coupling constants of peak multiplicities (J) have been expressed in Hz.
FT-IR (KBr): *v* (cm⁻¹) 3396 (OH), 2929 (CH), 1717 (**CO**-OH), 1612 (acetyl **CO**-N).

### Synthesis of (2-bromoisobutyryl)₇-maltotriosyl-N-acetyl-amino-dodecanoic acid

(2-bromoisobutyryl)₇-maltotriosyl-N-acetyl-amino-dodecanoic acid was synthesised according to a modified procedure of Stenzel-Rosenbaum et al. [*ref. cit.*]*.* Briefly, 1 g of maltotriosyl-N-acetyl-amino-dodecanoic acid (1.35 mmoles) were suspended in 20 mL of anhydrous chloroform containing 3.7 mL of triethylamine (26.7 mmoles). The suspension was chilled to 0°C, added of 3.3 mL of 2-bromo-isobutyryl-bromide (26.7 mmoles) in 30 min and left under stirring for 72 hours. The mixture was filtered and the organic solution treated three times with 30 mL cold water, three times with 20 mL of 0.1 N NaOH and three times with 30 mL of cold water. The organic phase was finally dried with anhydrous Na₂SO₄, filtered and concentrated under reduced pressure. The resulting red-brown solid was dissolved in 5 mL acetone and added of 100 mL cold water under vigorous stirring. Acetone was removed under reduced pressure and the aqueous suspension was lyophilised. The product yield was 1.47 g. The product was analysed by elemental analysis, ¹H-NMR and FT-IR.
Elemental analysis: C, 40.8%; H, 5.1%; N, 1.0%; Br, 30.4% (O, 22.7%) [calcd for (2-bromoisobutyryl)₇-maltotriosyl-N-acetyl-amino-dodecanoic acid (C₆₀H₉₂Br₇NO₂₅): C, 40.3%; H, 5.2%; N, 0.8%; Br, 31.3% (O, 22.4%)].
¹H NMR (CDCl₃): *δ* ppm 4.60-3.30 (bm, 18H of glycosyl moiety), 3.29 (m, 2H, λ-CH₂-), 2.15 (m, 2H, α-CH₂-), 1.96 (bs, 42H, CH₃), 1.94 (s, 3H, acetyl-CH₃), 1.59 (bm, 4H, ℩-CH₂- and β-CH₂-), 1.33-1.22 (bm, 14H, -CH₂- of alkyl chain).
FT-IR: *v* (cm⁻¹) 3437 (OH), 2932 (CH), 1743 (isobutyryl **CO**-O), 1619 (acetyl **CO-**N).

### Synthesis of methacryloyl-arginine

Methacryloyl-arginine was synthesised as described in example 1.

### Synthesis of tris[(2-pyridyl)methyl]amine (TPMA)

Tris[(2-pyridyl)methyl]amine (TPMA) was prepared as described in example 1.

### Synthesis of (arginyl)₇-maltotriosyl-N-acetyl-amino-dodecanoic acid

TPMA (1.31 g, 4.5 mmoles), CuBr (0.65 g, 4.5 mmoles) and ascorbic acid (7.93 mg, 0.045 mmoles) were dissolved in 5 mL of nitrogen purged DMSO and added to 15 mL of 0.1 g/mL methacryloyl-arginine solution (6.2 mmoles) in DMSO under stirring at 55°C. The solution was added of 3 mL of 0.27 g/mL of (2-bromoisobutyryl)₇-maltotriosyl-N-acetyl-amino-dodecanoic acid (0.45 mmoles) in nitrogen purged DMSO. The reaction mixture was maintained under nitrogen atmosphere for 12 hours and then was exposed to the air. After 30 min the solution was added dropwise to 200 mL of cold 1:1 acetone/diethyl ether mixture containing 1% acetic acid. The precipitate was desiccated under reduced pressure, redissolved in 5 mL of warm methanol and re-precipitated by dropping into 200 mL of the acetone/diethyl ether/acetic acid mixture. The product yield was 90% corresponding to 1.54 g.

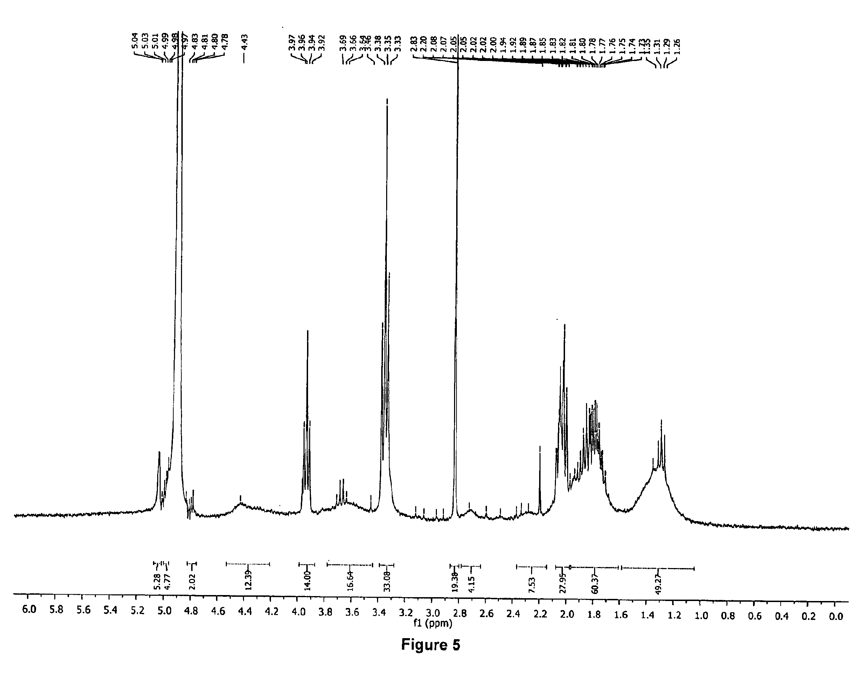

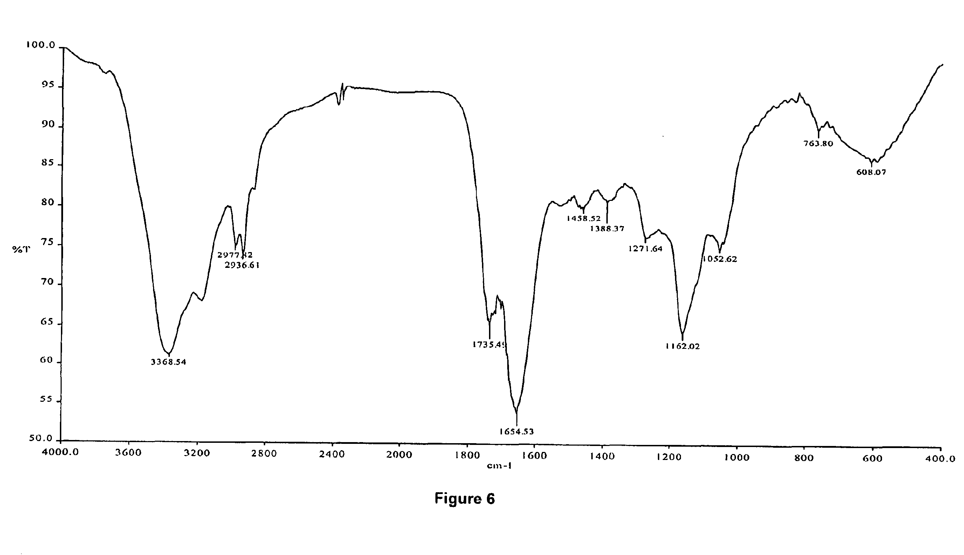

The arginine content in the final product was evaluated by Sakaguchi assay [*ref. cit.*]*.* The experimental data were referred to a calibration curve obtained with standard solutions of 0-0.6 µM guanidinium content (y = 7.3955x - 0.0386, R²=0.99). The final product was also analysed by elemental analysis, ¹H-NMR (FIG. 5), FT-IR.
Elemental analysis: found C, 39.3%; H, 5.9%; N, 10.0%; Br, 28.1% (O, 16.7%); [calcd for (arginyl)₇-N-acetyl-maltotriosylamino-dodecanoic acid HBr salt (C₁₃₀H₂₁₈Br₇N₂₉O₄₆·7HBr): C, 38.6%; H, 5.6%; N, 10.0%; Br, 27.6% (O, 18.2%)]. FT-IR: *v* (cm⁻¹) 3373 and 3182 [NHC(NH₂)₂⁺], 2938 (CH), 1735 (C=O), 1657 (C=N) (FIG 6).

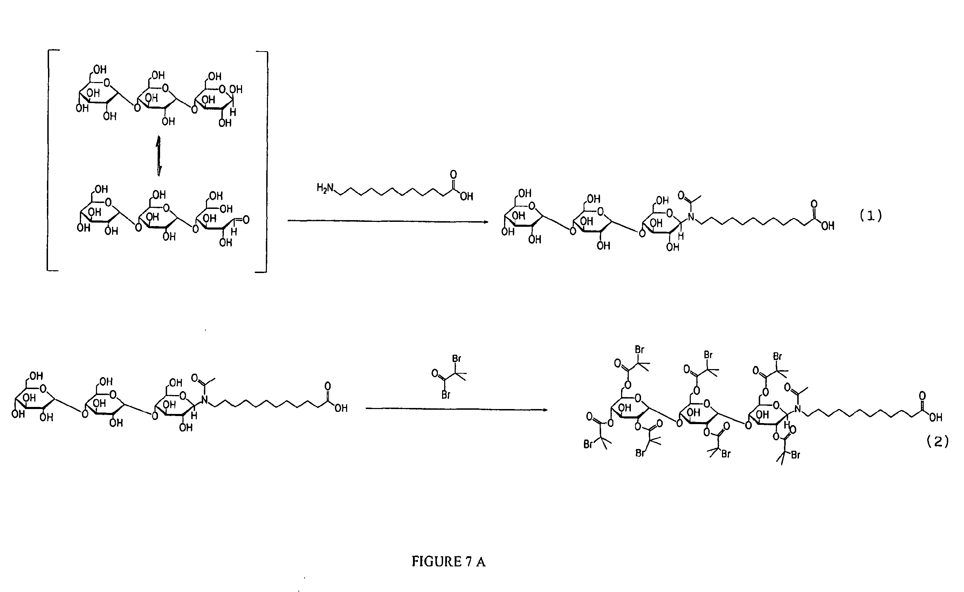

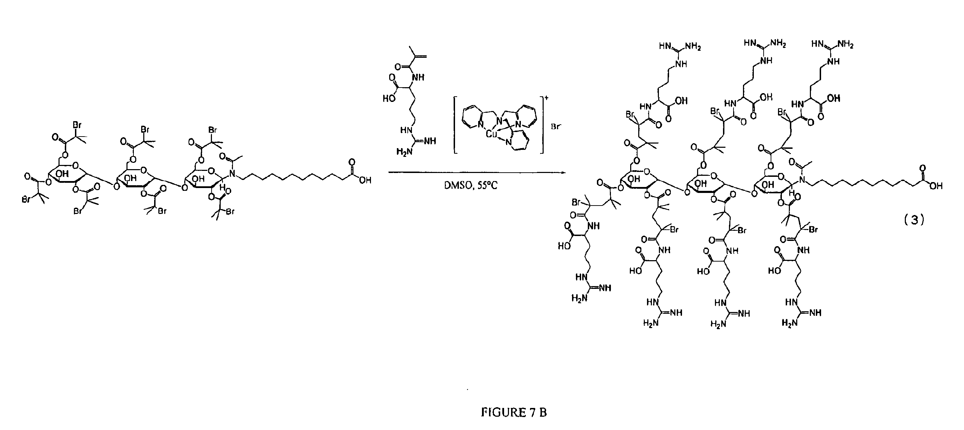

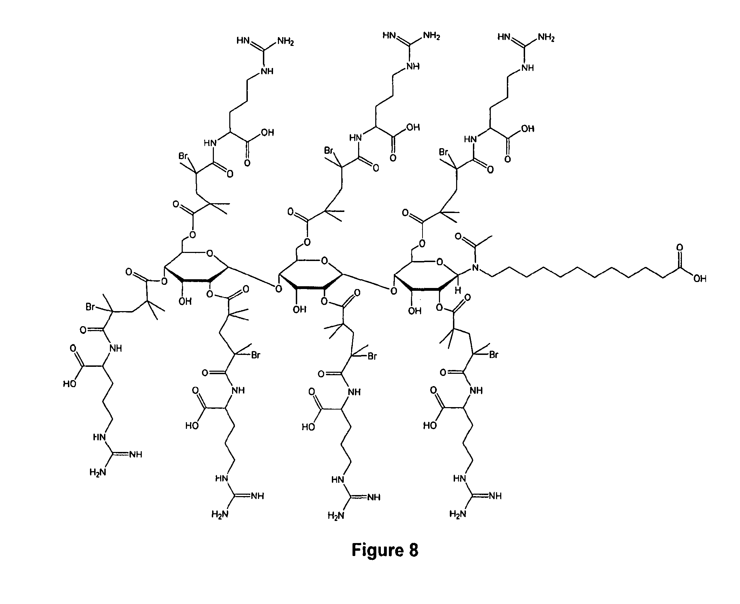

The synthetic scheme adopted the synthesis of (arginyl)₇-maltotriosyl-N-acetyl-amino-dodecanoic acid of is summarized in figures 7A-B and the chemical formula of the product are reported in figure 8.

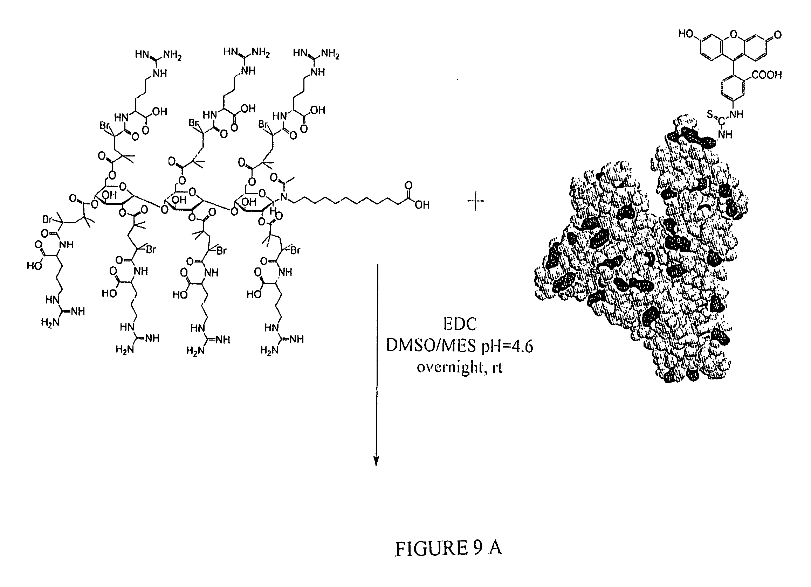

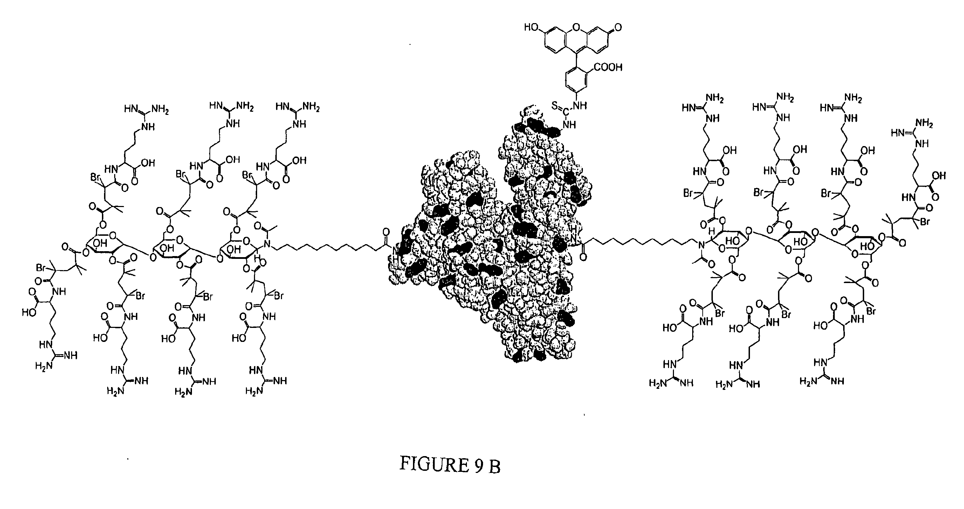

The epta-guanidyl derivative prepared as described in example 3 was conjugated to fluorescein labelled bovine serum albumin (FITC-BSA) used as cargo molecule model for testing the cell penetrating properties in cell cultures. The preparation of the (arginyl)₇-maltotriosyl-N-acetyl-amino-dodecanoyl-(FITC-BSA) is described in full detail in the following example 5. The synthetic scheme is summarized in figures 9A-B.

### Example 4. Synthesis of the corona-like (arginyl)₂₀-maltoheotaosyl-N-acetyl-poly(ethylene glycol)-carboxyl acid

### Synthesis of maltoheptaosyl-N-acetyl-poly(ethylene glycol)-carboxyl acid

NH₂PEG-COOH (147 mg, 0.043 mmoles) was dissolved in 4 mL of CH₃OH/0.5% acetic acid. The solution was thermostated at 60°C and added of 50 mg maltoheptaose (0.043 mmoles). After 24 hours, 41 µL of acetic anhydride (0.43 mmoles) were added to the mixture and the solution was maintained under stirring for 96 hours at room temperature. The volume was reduced to 0.5 mL under vacuum and the solution was added dropwise to 40 mL of diethyl ether. The precipitate was collected by filtration and desiccated under vacuum. The reaction yield was 187 mg. The product was also characterized by elemental analysis and ¹H-NMR (AMX 300 MHz, Bruker Spectrospin, Fallanden, Switzerland).
Elemental analysis: found C, 49.9%, H, 8.5%, N, 0.4% (O, 41.2%) [calcd for C₁₉₅H₃₇₇NO₁₁₃: C, 51.5%, H, 8.4%, N, 0.3% (O, 39.8%)].
¹H-NMR (D₂O): *δ* ppm 5.08 (bd, 6H anomeric H), 4.20-3.2 0 (bm, H of glycosyl moiety and λ-CH₂-), 3.38 (bs, 300H OCH₂CH₂), 2.40 (bs, 3H, acetyl-CH₃), 2.33 (t, 2H, α-CH₂-). NMR signals were indicated as singlet (s), broad singlet (bs), doublet (d), triplet (t), multiplet (m) and broad multiplet (bm).

### Synthesis of (2-bromoisobutyryl)₂₀-maltoheptaosyl-N-acetyl-poly(ethylene glycol)-carboxyl acid

(2-bromoisobutyryl)₂₀-maltoheptaosyl-N-acetyl-poly(ethylene glycol)-carboxyl acid was synthesised according to a modified procedure of Stenzel-Rosenbaum et al. [*ref. cit*.]. Briefly, 150 mg of *maltoheptaosyl-N-acetyl-poly(ethylene glycol)-carboxyl acid* (0.03 mmoles) were suspended in 8 mL of anhydrous chloroform containing 104 µL of triethylamine (0.75 mmoles). The suspension was chilled to 0°C, added of 111 µL of 2-bromo-isobutyryl-bromide (0.9 mmoles) in 30 min and left under stirring for 96 hours. The mixture was filtered and the organic solution treated three times with 15 mL cold water, three times with 10 mL of 0.1 N NaOH and three times with 15 mL of cold water. The organic phase was finally dried with anhydrous Na₂SO₄, filtered and concentrated under reduced pressure. The resulting red-brown solid was dissolved in 3 mL acetone and added of 100 mL cold water under vigorous stirring. Acetone was removed under reduced pressure and the aqueous suspension was lyophilised. The product yield was 142 mg. The product was analysed by elemental analysis, ¹H-NMR and FT-IR.
Elemental analysis: C, 42.1%; H, 7.7%; N, 0.6%; Br,22.1% (0,27.5%) [calcd for (2-bromoisobutyryl)₂₀-maltoheptaosyl-N-acetyl-poly(ethylene glycol)-carboxyl acid (C₂₇₅H₄₇₇Br₂₀NO₁₃₃): C,43.9%; H, 6.4%; N, 0.2%; Br, 21.2% (O, 28.3%)].

### Synthesis of methacryloyl-arginine

Methacryloyl-arginine was synthesised as described in example 1.

### Synthesis of tris[(2-pyridyl)methyl]amine (TPMA)

Tris[(2-pyridyl)methyl]amine (TPMA) was prepared as described in example 1. *Synthesis of (*arginyl*)₂₀*-*maltoheptaosyl-N*-*acetyl*-*poly(ethylene glycol)-carboxyl acid*

TPMA (76.6 mg, 0.264 mmoles), CuBr (37.9 mg, 0.264 mmoles) and ascorbic acid (0.18 mg, 0.67 10⁻³ mmoles) were dissolved in 3 mL of nitrogen purged DMSO and added to 26 mL of 10 mg/mL methacryloyl-arginine solution (1.06 mmoles) in DMSO under stirring at 55°C. The solution was added of 3 mL of 33 mg/mL of (2-bromoisobutyryl)₂₀-maltoheptaosyl-N-acetyl-poly(ethylene glycol)-carboxyl acid (0.013 mmoles) in nitrogen purged DMSO. The reaction mixture was maintained under nitrogen atmosphere for 12 hours and then was exposed to the air. After 30 min the solution was added dropwise to 50 mL of cold 1:1 acetone/diethyl ether mixture containing 1% acetic acid. The precipitate was desiccated under reduced pressure, redissolved in 5 mL of warm methanol and re-precipitated by dropping into 50 mL of the acetone/diethyl ether/acetic acid mixture. The product yield was 82% corresponding to 134 mg.

The arginine content in the final product was evaluated by Sakaguchi assay [*ref*. *cit*.]. The experimental data were referred to a calibration curve obtained with standard solutions of 0-0.6 µM guanidinium content (y = 7.3955x - 0.0386, R²=0.99).

### Example 5. Bovine serum albumin labelling with fluorescein and conjugation of (arginyl)₇-maltotriosyl-N-acetyl-amino-dodecanoic acid

Bovine serum albumin (BSA, 10 mg, 0.15 µmoles) was dissolved in 250 µl of 0.1 M borate buffer, pH 8.3, and added of 58 µl of 10 mg/mL of FITC (1.5 µmoles) in DMSO. The protein solution was left at room temperature under mild stirring for 1 hour in the dark and then was added of 15 µl of ethanolamine (249 µmoles). The FITC labelled protein was purified by fast protein liquid chromatography (FPLC) using a Superose 12 gel filtration column (Amersham-Pharmacia Biotech, Uppsala, Sweden) isocratically eluted with 0.5 mL/min of 100 mM phosphate buffer, pH 7.0. The fractions containing the fluorescent protein were pooled, lyophilized and analysed by RP-HPLC using a Jupiter C4 column (250x4.6mm, Phenomenex, Torrance, CA, USA) eluted with a gradient of H₂O/0.05% trifluoroacetic acid (A) and acetonitrile/0.05% trifluoroacetic acid (B) (0-3 min 5% B, 3-23 min from 5% to 55% B). The UV detector was set at 280 nm.

A DMSO solution (0.15 mL) containing 9.5 mg of (arginyl)₇-maltotriosyl-N-acetyl-amino-dodecanoic acid (2.72 µmoles) of example 2 was added to 1 mL of water containing 10 mg of EDC (52.16 µmoles). After 30 min the solution was slowly added to 3 mL of in 100 mM morpholino-ethan-sulfonic acid buffer (MES), pH 4.6. containing 19.8 mg of FITC-BSA (0.3 µmoles). The reaction mixture was maintained overnight under stirring in the dark and then ultrafiltered using Amicon System equipped with a 10 kDa cut-off membrane (Millipore Amicon, Bedford, MA, USA). The product was finally purified by ion exchange chromatography using a TSK-Gel Chelate-5PW column (Tosho Bioscience LLC, PA, USA) eluted with a gradient of 20 mM phosphate buffer, 0.1 M NaCl, pH 6.5 (Eluent A) and 20 mM phosphate buffer, 0.7 M NaCl, pH 6.5 (Eluent B): 0-3 min Eluent A 100%: 9 min Eluent A 0%; 12 min Eluent A 0%. The flow rate was 1 mUmin. The UV detector was set at 280 nm and the Fluorescence detector was set at 494 nm λ_{exc} and 520 nm λₑₘ. The elution volume corresponding to the bioconjugate was collected, ultrafiltered with a 10 kDa cut-off membrane to eliminate the salts and finally lyophilized. The final product was characterized by RP-HPLC as reported above. The number of (arginyl)₇-maltotriosyl-N-acetyl-amino-dodecanoyl moieties per FITC-BSA molecule was determined by Sakaguchi assay [*ref. cit*.] using FITC-BSA as reference.

The cell penetrating properties of the novel epta-arginyl derivative were investigated *in vitro* by cell incubation with (arginyl)₇-maltotriosyl-N-acetyl-amino-dodecanoyl-FITC-BSA and FITC-BSA as control.

### Example 6. Assay of the cell penetrating properties of the corona-like (arginyl)₇-maltotriosyl-N-acetyl-amino-dodecanoyl-FITC-BSA

### Cell lines and culture

The human MCF-7 breast adenocarcinoma and murine MC3T3-E1 embryonic fibroblast cell lines were growth in RPMI 1640 and DMEM medium, respectively, supplemented with 10% heated inactivated foetal bovine serum (FBS), 1% L-glutamine, 1% penicillin-streptomycin-amphotericin B mixture. All cells were maintained at 37 °C in humidified 5% CO₂ atmosphere and splitted every 2-3 day.

### Fluorescence analysis

MCF-7 and MC3T3-E1 cells were seeded in six-well plates (2.0×10⁶ cells/well). After 48 hours (90% confluence) the medium was removed the cells were incubated at 37°C with FITC-BSA or (arginyl)₇-maltotriosyl-N-acetyl-amino-dodecanoyl-FITC-BSA (0.2 µM in culture medium supplied with 10% FBS). After 30 min the medium was removed and the cells were washed three times with D-PBS and lysed in 1 mL with Triton X-100 (0.1%). The plates were gently shaken for 2 hours and the lysates were centrifuged at 12000 rpm (10625 g) for 5 min. The fluorescence intensity was measured at 520 nm by a FP-6500 Jasco Fluorescence Spectrophotometer (Kyoto, Japan) using Triton X-100 (0.1%) as blank. A similar procedure was carried out with unseeded plates in order to quantify the fluorescence intensity due to unspecific interaction of the fluorescein labelled BSA products to the tissue culture plastic material. The relative fluorescence/cell was determined on the basis of the BCA protein assay (Pierce/Celbio, Pero, Italy).

### Fluorescence-activated cell sorter (FACS) analysis

MCF-7 and MC3T3-E1 cells were seeded in six-well plates (1 × 10⁶ cells/well, 1.8 mL culture medium/well). After 48 hours, the medium was removed and the cells were rinsed with 2.0 mL of D-PBS and incubated with FITC-BSA or (arginyl)₇-maltotriosyl-N-acetyl-amino-dodecanoyl-FITC-BSA (0.2 µM in culture medium supplied with 10% FBS) as described above. After 30 min incubation, the cells were washed three times with D-PBS and treated with trypsin-EDTA. Trypsinized cells were recovered by centrifugation at 1500 rpm (166 g) for 2 min and washed twice with D-PBS. The cells were fixed with 1% (w/v) paraformaldehyde in phosphate buffer and stored at 4°C protected from light overnight. Afterwards, the cell samples (100,000 cells in average count) were analysed by a FACSCalibur flow cytometer using CellQuest software package (BD Bioscience, Heidelberg, Germany) and gated using forward *versus* side scatter to exclude debris and dead cells.

### Confocal microscopic observation

MCF-7 and MC3T3-E1 cells were plated on four-well chamber slides at a density of 1 × 10⁵ cells for chamber and incubated at 37 °C in humidified 5% CO₂ atmosphere. After 24 hours adhesion, the cells were incubated with FITC-BSA or (arginyl)₇-maltotriosyl-N-acetyl-amino-dodecanoyl-FITC-BSA (0.2 µM in culture medium supplemented with 10% FBS) as described above. After 30 min incubation at 37 °C, the cells were extensively washed with D-PBS and fixed with 1% (w/v) paraformaldehyde in phosphate buffer for 1 hour at 4°C. Afterwards, fixed cells were rinsed twice with D-PBS and the samples were mounted with Vectashield^{®} containing 1.5 µg/mL DAPI and kept at 4 °C and protected from light until microscopic examination. Confocal imaging was performed using a Leica TCS SP5 confocal laser-scanning microscope (Leica Microsystems, Mannheim, Germany) equipped with a Leica HCX PL APO 63x/1.40 oil immersion objective. The Blue argon laser was set at 351 nm (blue) and 488 nm (green) to produce the excitation wavelength for DAPI and fluorescein, respectively.

### Spectrofluorimetric analysis

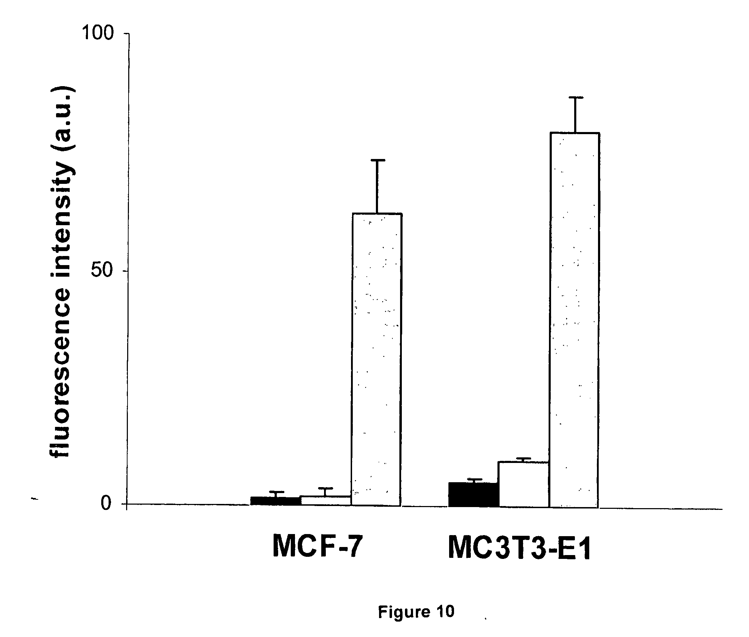

The cell associated relative fluorescence intensities were examined after MCF-7 and MC3T3-E1 FITC-BSA incubation with (arginyl)₇-maltotriosyl-N-acetyl-amino-dodecanoyl-FITC-BSA and cell lysis with Triton X-100. Parallel studies were carried out using untreated cells to assess the basal cell fluorescence (negative control). The experimental values reported in figure 10 show that a similar behaviour is obtained with MCF-7 and MC3T3-E1 cells. The relative fluorescence obtained after MCF-7 incubation with FITC-BSA was similar to the basal florescence obtained with untreated cells (2.19±1.52 and 1.85±1.06, respectively), while a negligible increase of cell fluorescence was observed with MC3T3-E1 incubated with FITC-BSA as compared to the untreated cells (9.62±0.64 and 5.17±0.51, respectively). On the contrary, the fluorescence intensity values of MCF-7 and MC3T3-E1 cells incubated with (arginyl)₇-maltotriosyl-N-acetyl-amino-dodecanoyl-FITC-BSA were remarkably higher as compared to that obtained with FITC-BSA treated cells. The MCF-7 and MC3T3-E1 incubation with (arginyl)₇-maltotriosyl-N-acetyl-amino-dodecanoyl-FITC-BSA resulted in about 34 and 9 fold fluorescence increase, respectively, as compared to the fluorescence obtained after cell incubation with FITC-BSA.

### Flow cytometry analysis

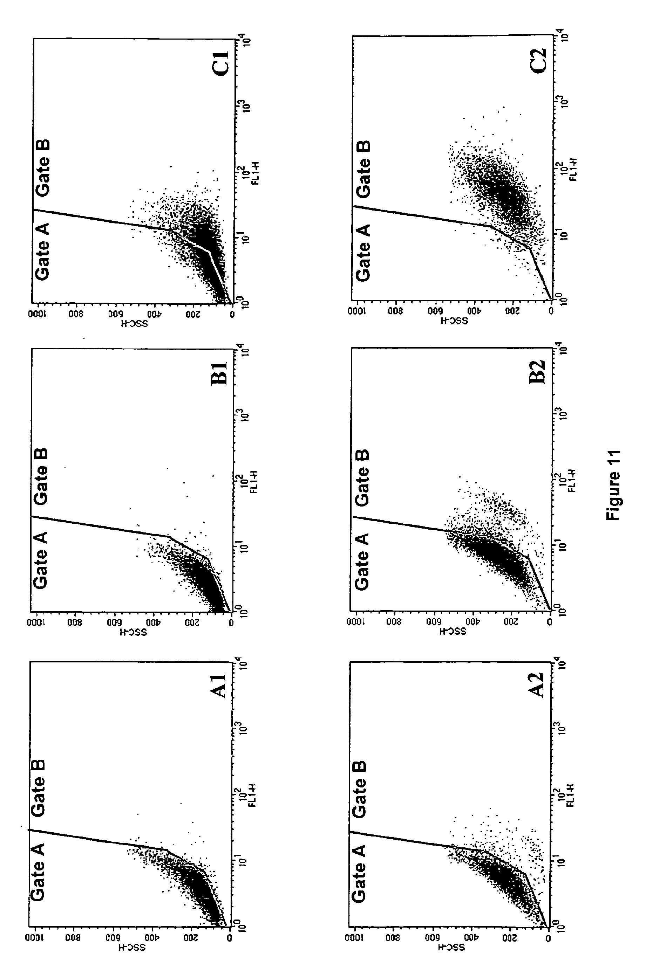

The dot plots of side scatter (SSC-H) *versus* fluorescence intensity (FL1-H) obtained by fluorescence activated cell sorting are reported in figure 11. The plots obtained from MCF-7 and MC3T3-E1 incubated with FITC-BSA (panels A2 and B2, respectively) show that the amount of cells with of higher fluorescence than the basal value is similar to that displayed by the untreated cells (panels A1 and B1). The percentage of FITC-BSA treated MCF-7 and MC3T3-E1 cells with higher fluorescence as compared to the basal fluorescence value were 0.2% and 10%, respectively. The dot plots reported in panels A3 and B3 show that incubation of (arginyl)₇-maltotriosyl-N-acetyl-amino-dodecanoyl-FITC-BSA increases significantly the cell associated fluorescence. About 25% of the (arginyl)₇-maltotriosyl-N-acetyl-amino-dodecanoyl-FITC-BSA treated MCF-7 cells and 95% of epta-arginyl-FITC-BSA treated MC3T3-E1 cells display higher fluorescence as compared to the basal values.

### Confocal microscopy

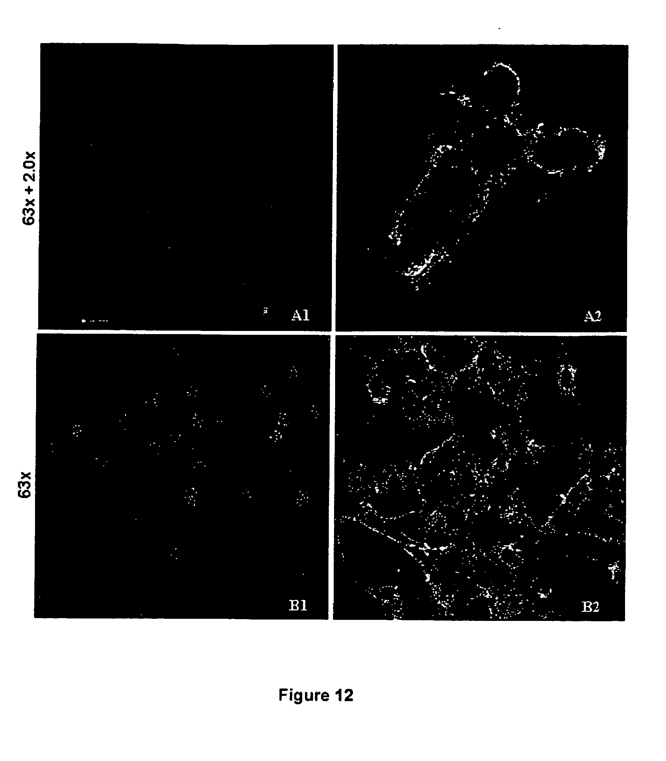

The confocal images obtained with MCF-7 and MC3T3-E1 cells incubated with FITC-BSA and (arginyl)₇-maltotriosyl-N-acetyl-amino-dodecanoyl-FITC-BSA are reported in figure 12. The images obtained with FITC-BSA treated MCF-7 and MC3T3-E1 cells (A1 and B1, respectively) show the intense blue fluorescence (dark grey on a black background in the figure) corresponding to the DAPI stained nuclei. No green fluorescence associated to FITC could be observed in these samples indicating that FITC-BSA was neither cell surface adsorbed nor cell internalized. On the contrary, the images obtained with (arginyl)₇-maltotriosyl-N-acetyl-amino-dodecanoyl-FITC-BSA treated MCF7 and MC3T3-E1 cells (A2 and B2, respectively) show intense green punctuated fluorescence in the cytosol (light grey surrounding the dark grey in the figure). The image elaboration did not revealed detectable green fluorescence in the nuclei.

### Example 7. Citoxicity test of (arginyl)₇-maltotriosyl-N-acetyl-amino-dodecanoic acid

MCF-7 and MC3T3-E1 cells were plated in 96-well flat bottomed microassay plates (1 × 10⁴ cells/well) and grown for 24 hours. After complete adhesion, (arginyl)₇-maltotriosyl-N-acetyl-amino-dodecanoic acid or 2 kDa polyethylenimine (PEI) as control were added to the culture media at increasing concentrations: from 0 to 100 µM. The cells were incubated for 24 hours at 37°C and then the medium was discarded by aspiration and replaced with 200 µl of fresh medium. The cells were grown for further 48 hours. Twenty microliters of 5 mg/mL of 3-(4,5-dimethylthiazol-2-yl)-2,5-diphenyltetrazolium bromide (MTT) in D-PBS were added to each well. After 3 hours the MTT containing medium was removed and 200 µl of DMSO were added to each well in order to dissolve the formazan crystal formed by alive cells (Mosmann T 1983). The plates were gently shaken for 30 min and absorbance was measured at 570 nm with a reference wavelength of 630 nm. The cell viability was expressed as a percent viability of treated cells against untreated cells used as control.

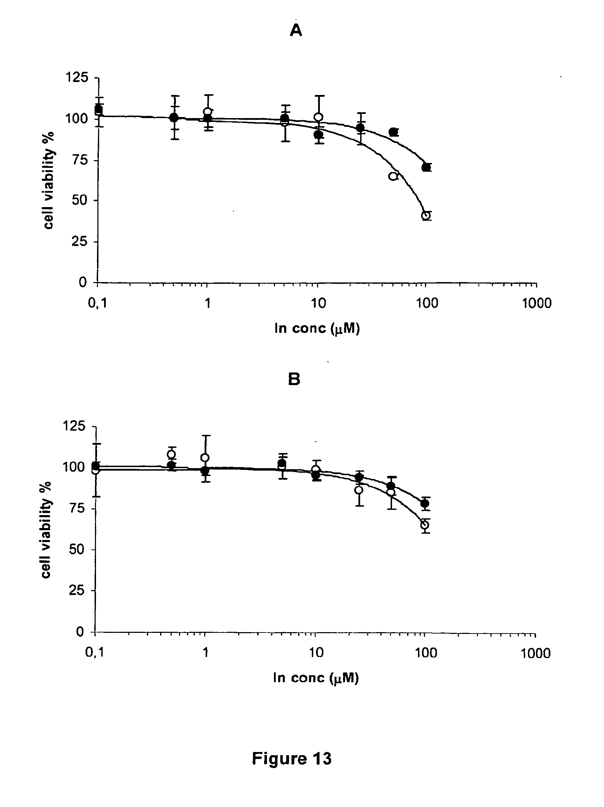

The cytotoxicity profiles reported in figure 13 show that the (arginyl)₇-maltotriosyl-N-acetyl-amino-dodecanoic acid has a very low toxicity towards both the examined cell lines as compared to PEI. The IC50 calculated for PEI was 40 and 30 µM in the case of MCF-7 and MC3T3-E1, respectively, while the (arginyl)₇-maltotriosyl-N-acetyl-amino-dodecanoic acid IC50 obtained with both cell lines was higher than 100 µM. Higher (arginyl)₇-maltotriosyl-N-acetyl-amino-dodecanoic acid concentrations could not be examined because of the solubility limit of this product in the medium used for the cell viability assay.

### References

- Green M, Loewenstein PM (1988). Autonomous functional domain of chemically synthesized human immunodeficiency virus tat trans-activator protein. Cell. 55:1179-1188.
- Goutam B, Ock-Youm J, Woo Sirl L, Dong-Chan K. Kyong-Tai K, Suho L, Sunghoer C and Sung-Kee C (2008). Novel guanidine-containing molecular transporters based on lactose scaffolds: lipophilicity effect on the intracellular organellar selectivity. Chem. Eur. 14:9161-9168.
- Kalderon D, Roberts BL, Richardson WD, Smith AE (1984). A short amino acid sequence able to specify nuclear location. Cell. 39:499-509.
- Futaki S, Suzuki T, Ohashi W, Yagami T, Tanaka S, Ueda K, Sugiura Y (2001). Arginine-rich peptides. An abundant source of membrane-permeable peptides having potential as carriers for intracellular protein delivery. J Biol Chem. 276:5836-5840.
- Fonseca S, Pereira MP, Kelley SO (2009). Recent advances in the use of cell-penetrating peptides for medical and biological applications. Adv Drug Deliver Rev. 61:953-964.
- Fisher AA, Ye D, Sergueev DS, Fisher MH, Shaw BR, Juliano RL (2002). Evaluating the specificity of antisense oligonucleotide conjugates. A DNA array analysis. J Biol Chem. 277:22980-22984.
- Veldhoen S, Laufer SD, Restle T (2008). Recent developments in Peptide-based nucleic Acid delivery. Int J Mol Sci. 9:1276-1320.
- Bonny C, Oberson A, Negri S, Sauser C, Schorderet DF (2001). Cell-permeable peptide inhibitors of JNK: novel blockers of beta-cell death. Diabetes. 50:77-82.
- Peitz M, Pfannkuche K, Rajewsky K, Edenhofer F (2002). Ability of the hydrophobic FGF and basic TAT peptides to promote cellular uptake of recombinant Cre recombinase: a tool for efficient genetic engineering of mammalian genomes. Proc Natl Acad Sci U S A. 99:4489-4494.
- Säälik P, Elmquist A, Hansen M, Padari K, Saar K, Viht K, Langel U, Pooga M (2004). Protein Cargo Delivery Properties of Cell-Penetrating Peptides. A Comparative Study. Bioconjugate Chem. 15:1246-1253.
- Torchilin VP (2008). Tat peptide-mediated intracellular delivery of pharmaceutical nanocarriers. Adv Drug Deliver Rev. 60:548-558.
- Lewin M, Carlesso N, Tung CH, Tang XW, Cory D, Scadden DT, Weissleder R (2000). Tat peptide-derivatized magnetic nanoparticles allow in vivo tracking and recovery of progenitor cells. Nat Biotechnol. 18:410-414.
- Medintz IL, Pons T, Delehanty JB, Susumu K, Brunel FM, Dawson PE, Mattoussi H (2008). Intracellular Delivery of Quantum Dot-Protein Cargos Mediated by Cell Penetrating Peptides. Bioconjugate Chem. 19:1785-1795.
- Tréhin R, Merkle HP (2004). Chances and pitfalls of cell penetrating peptides for cellular drug delivery. Eur J Pharm Biopharm. 58:209-223.
- Stenzel-Rosenbaum MH, Davis TP, Chen V, Fane AG (2001), Macromolecules. 34:5433-5438.
- Tyeklàr Z, Jacobson RR, Wei N, Murthy NN, Zubieta J, Karlin KD (1993), J Am Chem Soc. 115:2677-2689.
- Sakaguchi S (1925). A new color reaction of protein and arginine. J Biochem. 5:25-31.
- Mosmann T (1983), J Immunol Methods. 65:55-63.

## Claims

1. A molecule consisting of a (guanidyl)-oligosaccharidic derivative represented by the general formula (I) 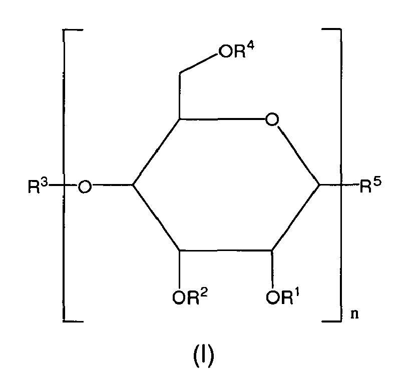 wherein:
R¹, R², R³, R⁴, equal or different from each other, are:
- a hydrogen -H;
- a linker of formula -CO-R^{I}-X, where R^{I} is a C₂-C₃ alkyl branched chain and where X is a halogen; or
- a guanidine terminating group of formula -CO-R^{I}-[CH₂C(CH₃)-CO-NH-R^{II}-NHC(NH₂)=NH]-X, where R^{I} and X have the same meanings previously mentioned for the linker, and R" is a linear or branched C₄-C₈ alkyl chain optionally bearing a -OH or a -COOH group,
with the proviso that R¹, R², R³, R⁴ represent, independently one from the other, at least one guanidine terminating group of formula -CO-R^{I}-[CH₂C(CH₃)-CO-NH-R^{II}-NHC(NH₂)=NH]-X;
R⁵ is:
- a spacer -(CH₃CO)N-R^{III}-Y, where R^{III} is a linear or branched C₈-C₂₂ alkyl chain or an oligo(ethylene glycol) chain derivative of formula - [CH₂CH₂O]ₘ-(CH₂)ₚ where m is an integer selected in the range from 2 to 136 and p is an integer selected from the range from 2 to 6, and Y is a terminating group selected from the carboxyl-, amino-, hydroxyl- or thiol- groups;
n is an integer ranging from 2 to 8,
and salts thereof.

2. The molecule consisting of a (guanidyl)-oligosaccharidic derivative according to claim 1, wherein the oligosaccharide is selected from the group consisting of lactose, mannose, cellobiose, maltose, isomaltose, maltotriose, maltotetraose, maltopentose, maltohexaose, maltohepatose, maltooctaose.

3. The molecule consisting of a (guanidyl)-oligosaccharidic derivative according to claim 1, wherein the oligosaccharide is selected from the group consisting of lactose, maltose and maltotriose.

4. The molecule consisting of a (guanidyl)-oligosaccharidic derivative according to claim 1, wherein R¹, R², R³, R⁴ are, independently one from the other, at least 2 and up to 20 guanidine terminating groups of formula -CO-R^{I}-[CH₂C(CH₃)-CO-NH-R^{II}-NHC(NH₂)=NH]-X.

5. The molecule consisting of a (guanidyl)-oligosaccharidic derivative according to claim 4, wherein the guanidine terminating group of formula -CO-R^{I}-[CH₂C(CH₃)-CO-NH-R^{II}-NHC(NH₂)=NH]-X are from 4 to 7 when the oligosaccharidic core is a disaccharide, and from 2 to 20, when the oligosaccharidic core is a heptasaccharide.

6. The molecule consisting of a (guanidyl)-oligosaccharidic derivative according to claim 1, wherein R¹, R², R³, R⁴ are all a guanidine terminating group of formula -CO-R^{I}-[CH₂C(CH₃)-CO-NH-R^{II}-NHC(NH₂)=NH]-X.

7. The molecule consisting of a (guanidyl)-oligosaccharidic derivative according to claim 1, wherein in R¹, R², R³, R⁴ R^{I} is an isopropyl group and X is Cl or Br.

8. The molecule consisting of a (guanidyl)-oligosaccharidic derivative according to claim 1, wherein the linker of formula -CO-R^{I}-X is the 2-bromoisobutyryl group.

9. The molecule consisting of a (guanidyl)-oligosaccharidic derivative according to claim 1, wherein when R¹, R², R³ are, independently one from the other, guanidine terminating groups of formula -CO-R^{I}-[CH₂C(CH₃)-CO-NH-R^{II}-NHC(NH₂)=NH]-X R" is a C₄ alkyl chain bearing a -COOH group.

10. The molecule consisting of a (guanidyl)-oligosaccharidic derivative according to claim 1, wherein in R⁵ R^{III} is a C₁₀-C₁₈ alkyl chain or an oligo(ethylene glycol) chain -[CH₂CH₂O]ₘ having a molecular weight comprised from 200 to 3400 Da and m is from 5 to 77 and Y is a terminating functional group selected from -COOH or -NH₂.

11. The molecule consisting of a (guanidyl)-oligosaccharidic derivative according to claim 1, wherein the spacer R⁵ is N-acetyl-12-aminododecanoyl acid.

12. The molecule consisting of a (guanidyl)-oligosaccharidic derivative according to one of the claims from 1 to 11 for use as carriers for intracellular delivery of macromolecules or supramolecular colloidal structures physically or covalently conjugated thereto.

13. The molecule consisting of a (guanidyl)-oligosaccharidic derivative according to the claim 12 for use as carriers of macromolecules selected from proteins, oligonucleotides, siRNA, genes, polymer therapeutics, dendrimers.

14. The molecule consisting of a (guanidyl)-oligosaccharidic derivative according to the claim 12 for use as carriers of supramolecular colloidal structures selected from supramolecular cargoes, liposomes, micelles, nanoparticles.

15. A method for preparing a molecule consisting of a (guanidyl)-oligosaccharidic derivative according to one of the claims 1 to 11 comprising at least the steps of:
- oligosaccharides derivatization on the anomeric carbon of the terminal glycosidic unit with a spacer of formula -HN-R^{III}-Y, where R^{III} is a linear or branched C₈-C₂₂ alkyl chain or an oligo(ethylen glycol) chain derivative of formula -[CH₂CH₂O]ₘ-(CH₂)ₚ where m is an integer selected in the range from 2 to 136 and p is an integer selected from the range from 2 to 6, and Y is a terminating group selected from the carboxyl-, amino-, hydroxyl- or thiol- groups;
- N-acetylation of the oligoglycosylamino-derivative obtained;
- esterification of oligosaccharide hydroxyl groups with a linker of formula -CO-R^{I}-X, where R^{I} is a C₂-C₃ alkyl branched chain and X is a halogen;
- conjugation of guanidyl residue of formula -[CH₂C(CH₃)-CO-NH-R^{II}-NHC(NH₂)=NH]-X, where R^{II} is a linear or branched C₄-C₈ alkyl chain optionally bearing a -OH or a -COOH group, to the linkers previously introduced on the hydroxyl groups of the oligosaccharidic backbone via atom transfer radical polymerization (ATRP).

## Patentansprüche

1. Molekül bestehend aus einem (Guanidyl)-oligosaccharidischem Derivat, welches durch die folgende Formel (I) dargestellt ist: 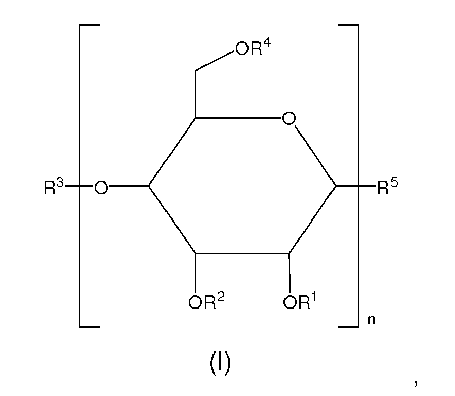 worin:
R¹, R², R³ und R⁴ gleich sind oder voneinander verschieden sind und sind:
- ein Wasserstoff -H;
- eine Verbindungsgruppe der Formel -CO-R^{I}-X, worin R^{I} eine verzweigte C₂-C₃ Alkylkette ist und worin X ein Halogen ist; oder
- eine Guanidin Endgruppe der Formel -CO-R^{I}-[CH₂C(CH₃)-CO-NH-R^{II}-NHC(NH₂)=NH]-X, worin R^{I} und X die gleiche Bedeutung haben, wie zuvor für die Verbindungsgruppe erwähnt und worin R^{II} eine lineare oder verzweigte C₄-C₈-Alkylkette ist, welche optional eine-OH- oder eine -COOH-Gruppe trägt, ist,
mit der Maßgabe, dass R¹, R², R³ und R⁴ unabhängig voneinander wenigstens eine Guanidin-Endgruppe der Formel -CO-R^{I}-[CH₂C(CH₃)-CO-NH-R^{II}-NHC(NH₂)=NH]-X darstellen;
R₅ ist:
- ein Abstandhalter -(CH₃CO)N-R^{III}-Y, worin R^{III} eine lineare oder verzweigte C₈-C₂₂ Alkylkette oder ein Derivat einer Oligo(ethylenglykol)kette der Formel -[CH₂CH₂-O]ₘ-(CH₂)ₚ darstellt, worin m eine ganze Zahl ist, die aus dem Bereich von 2 bis 136 ausgewählt ist, und p eine ganze Zahl ist, die aus dem Bereich von 2 bis 6 ausgewählt ist, und Y eine Endgruppe ist, die aus Carboxyl-, Amino-, Hydroxy- oder Thiolgruppen ausgewählt ist,
n eine ganze Zahl aus dem Bereich von 2 bis 8 ist,
und Salzen davon.

2. Molekül bestehend aus einem (Guanidyl)-oligosaccharidischem Derivat nach Anspruch 1, wobei das Oligosaccharid aus der Gruppe bestehend aus Lactose, Mannose, Cellobiose, Maltose, Isomaltose, Maltotriose, Maltotetraose, Maltopentose, Maltohexaose, Maltoheptaose, Maltooctaose ausgewählt ist.

3. Molekül bestehend aus einem (Guanidyl)-oligosaccharidischem Derivat nach Anspruch 1, wobei das Oligosaccharid aus der Gruppe bestehend aus Lactose, Maltose und Maltotriose ausgewählt ist.

4. Molekül bestehend aus einem (Guanidyl)-oligosaccharidischem Derivat nach Anspruch 1, wobei R¹, R², R³ und R⁴ unabhängig voneinander wenigstens 2 und bis zu 20 Guanidin-Endgruppen der Formel -CO-R^{I}-[CH₂C(CH₃)-CO-NH-R^{II}-NHC(NH₂)=NH]-X sind.

5. Molekül bestehend aus einem (Guanidyl)-oligosaccharidischem Derivat nach Anspruch 4, wobei von der Guanidin-Endgruppe der Formel -CO-R^{I}-[CH₂C(CH₃)-CO-NH-R^{II}-NHC(NH₂)=NH]-X 4 bis 7 vorliegen, wenn der Oligosaccharidkern ein Disaccharid ist, und 2 bis 20 vorliegen, wenn der Oligosaccharidkern ein Heptasaccharid ist.

6. Molekül bestehend aus einem (Guanidyl)-oligosaccharidischem Derivat nach Anspruch 1, wobei alle R¹, R², R³ und R⁴ Guanidin-Endgruppen der Formel -CO-R^{I}-[CH₂C(CH₃)-CO-NH-R^{II}-NHC(NH₂)=NH]-X sind.

7. Molekül bestehend aus einem (Guanidyl)-oligosaccharidischem Derivat nach Anspruch 1, wobei in R¹, R², R³ und R⁴ R^{I} eine Isopropylgruppe ist und X Cl oder Br ist.

8. Molekül bestehend aus einem (Guanidyl)-oligosaccharidischem Derivat nach Anspruch 1, wobei die Verbindungsgruppe der Formel -CO-R^{I}-X die 2-Bromoisobutyrylgruppe ist.

9. Molekül bestehend aus einem (Guanidyl)-oligosaccharidischem Derivat nach Anspruch 1, wobei, wenn R¹, R² und R³ unabhängig voneinander Guanidin-Endgruppen der Formel -CO-R^{I}-[CH₂C(CH₃)-CO-NH-R^{II}-NHC(NH₂)=NH]-X sind, R^{II} eine -COOH-Gruppe tragende C₄-Alkylkette ist.

10. Molekül bestehend aus einem (Guanidyl)-oligosaccharidischem Derivat nach Anspruch 1, wobei in R⁵ R^{III} eine C₁₀-C₁₈ Alkylkette ist oder eine Oligo(ethylenglykol)kette ist, die ein Molekulargewicht von 200 bis 3400 Da umfasst, und m 5 bis 77 ist sowie Y eine funktionelle Endgruppe ist, die aus -COOH oder -NH₂ ausgewählt ist.

11. Molekül bestehend aus einem (Guanidyl)-oligosaccharidischem Derivat nach Anspruch 1, wobei der Abstandshalter R⁵ N-Acetyl-12-aminododecanoylsäure ist.

12. Molekül bestehend aus einem (Guanidyl)-oligosaccharidischem Derivat nach einem der Ansprüche 1 bis 11 zur Verwendung als Träger für die intrazelluläre Übertragung von Makromolekülen oder von supramolekularen kolloidalen Strukturen, die physikalisch oder kovalent daran konjugiert sind.

13. Molekül bestehend aus einem (Guanidyl)-oligosaccharidischem Derivat nach Anspruch 12 zur Verwendung als Träger für die intrazelluläre von Makromolekülen, die aus Proteinen, Oligonukleotiden, siRNA, Genen, polymeren Therapeutika und Dendrimeren ausgewählt sind.

14. Molekül bestehend aus einem (Guanidyl)-oligosaccharidischem Derivat nach Anspruch 12 zur Verwendung als Träger für supramolekulare kolloidale Strukturen, die aus supramolekularen Gütern, Liposomen, Mizellen und Nanopartikeln ausgewählt sind.

15. Verfahren zur Herstellung eines Moleküls bestehend aus einem (Guanidyl)-oligosaccharidischem Derivat nach einem der Ansprüche 1 bis 11, umfassend wenigstens die Schritte:
- Derivatisieren der Oligosaccharide am anomeren Kohlenstoff der terminalen glycosidischen Einheit mit einem Abstandshalter der Formel
- NH-R^{III}-Y, worin R^{III} eine lineare oder verzweigte C₈-C₂₂-Alkylkette oder ein Derivat von Oligo(ethylenglykol)ketten der Formel -[CH₂CH₂O]ₘ-(CH₂)ₚ ist, worin m eine aus dem Bereich vom 2 bis 136 ausgewählte ganze Zahl ist und Y eine Endgruppe ist, die aus Carboxyl-, Amino-, Hydroxy- oder Thiolgruppen ausgewählt ist;
- N-Acetylieren des erhaltenen Oligoglycosylaminoderivats;
- Verestern der Oligosaccharid-Hydroxygruppen mit einer Verbindungsgruppe der Formel -CO-R^{I}-X, worin R^{I} eine verzweigte C₂-C₃-Alkylkette und X ein Halogen ist;
- Konjugieren eines Guanidylrestes der Formel -[CH₂C(CH₃)-CO-NH-R^{II}-NHC(NH₂)=NH]-X, worin R^{II} eine lineare oder verzweigte C₄-C₈-Alkylkette ist, welche optional eine -OH- oder eine -COOH-Gruppe trägt, an die Verbindungsgruppen, welche zuvor an die Hydroxylgruppen des oligosaccharidischen Rückgrats durch radikalische Atomtransferpolymerisation (ATRP) eingeführt wurden.

## Revendications

1. Molécule constituée d'un dérivé (guanidyl)-oligosaccharidique représentée par la formule générale (I) 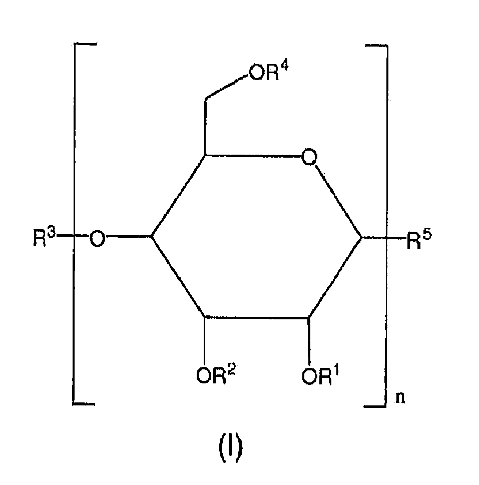 dans laquelle :
R¹, R², R³, R⁴, égaux ou différents les uns des autres, sont :
- un hydrogène -H ;
- un lieur de formule -CO-R'-X, où R' est une chaîne ramifiée d'alkyle en C₂-C₃ et où X est un halogène ; ou
- un groupe de terminaison guanidine de formule CO-R'-[CH₂C(CH₃)-CO-NH-R"-NHC(NH₂)=NH]-X, où R' et X ont les mêmes significations que susmentionnées pour le lieur, et R" est une chaîne alkyle en C₄-C₈ linéaire ou ramifiée portant éventuellement un groupe -OH ou un groupe -COOH,
à condition que R¹, R², R³, R⁴ représentent, indépendamment les uns des autres, au moins un groupe de terminaison guanidine de formule -CO-R'-[CH₂C(CH₃)-CO-NH-R"-NHC(NH₂)=NH]-X ;
R⁵ est :
- un espaceur -(CH₃CO)N-R"'-Y, où R"' est une chaîne alkyle en C₈-C₂₂ linéaire ou ramifiée ou un dérivé de chaîne oligo(éthylène glycol) de formule-[CH₂CH₂O]ₘ-(CH₂)ₚ où m est un entier choisi dans la plage de 2 à 136 et p est un entier choisi dans la plage de 2 à 6, et Y est un groupe de terminaison choisi parmi les groupes carboxyle, amino, hydroxyle ou thiol ;
n est un entier allant de 2 à 8,
et ses sels.

2. Molécule constituée d'un dérivé (guanidyl)-oligosaccharidique selon la revendication 1, dans laquelle l'oligosaccharide est choisi dans le groupe constitué de lactose, mannose, cellobiose, maltose, isomaltose, maltotriose, maltotétraose, maltopentose, maltohexaose, maltohépatose, maltooctaose.

3. Molécule constituée d'un dérivé (guanidyl)-oligosaccharidique selon la revendication 1, dans laquelle l'oligosaccharide est choisi dans le groupe constitué de lactose, maltose et maltotriose.

4. Molécule constituée d'un dérivé (guanidyl)-oligosaccharidique selon la revendication 1, dans laquelle R¹, R², R³, R⁴ représentent, indépendamment les uns des autres, au moins 2 et jusqu'à 20 groupes de terminaison guanidine de formule -CO-R'-[CH₂C(CH₃)-CO-NH-R"-NHC(NH₂)=NH]-X.

5. Molécule constituée d'un dérivé (guanidyl)-oligosaccharidique selon la revendication 4, dans laquelle le groupe de terminaison guanidine de formule -CO-R'-[CH₂C(CH₃)-CO-NH-R''-NHC(NH₂)=NH]-X est de 4 à 7 lorsque le noyau oligosaccharidique est un disaccharide, et de 2 à 20, lorsque le noyau oligosaccharidique est un heptasaccharide.

6. Molécule constituée d'un dérivé (guanidyl)-oligosaccharidique selon la revendication 1, dans laquelle R¹, R², R³, R⁴ sont tous un groupe de terminaison guanidine de formule -CO-R'-[CH₂C(CH₃)-CO-NH-R"-NHC(NH₂)=NH]-X.

7. Molécule constituée d'un dérivé (guanidyl)-oligosaccharidique selon la revendication 1, dans laquelle dans R¹, R², R³, R⁴, R' est un groupe isopropyle et X est Cl ou Br.

8. Molécule constituée d'un dérivé (guanidyl)-oligosaccharidique selon la revendication 1, dans laquelle le lieur de formule -CO-R'-X est le groupe 2-bromoisobutyryle.

9. Molécule constituée d'un dérivé (guanidyl)-oligosaccharidique selon la revendication 1, dans laquelle lorsque R¹, R², R³ sont, indépendamment les uns des autres, des groupes de terminaison guanidine de formule -CO-R'-[CH₂C(CH₃)-CO-NH-R"-NHC(NH₂)=NH]-X, R" est une chaîne alkyle en C₄ portant un groupe -COOH.

10. Molécule constituée d'un dérivé (guanidyl)-oligosaccharidique selon la revendication 1, dans laquelle dans R⁵, R"' est une chaîne alkyle en C₁₀-C₁₈ ou une chaîne oligo(éthylène glycol) -[CH₂CH₂O]ₘ ayant une masse moléculaire comprise entre 200 et 3400 Da et m est de 5 à 77 et Y est un groupe fonctionnel de terminaison choisi entre -COOH et -NH₂.

11. Molécule constituée d'un dérivé (guanidyl)-oligosaccharidique selon la revendication 1, dans laquelle l'espaceur R⁵ est un acide N-acétyl-12-aminododécanoylique.

12. Molécule constituée d'un dérivé (guanidyl)-oligosaccharidique selon l'une des revendications 1 à 11 pour utilisation en tant que support pour l'administration intracellulaire de macromolécules ou structures colloïdales supramoléculaires conjuguées physiquement ou de manière covalente à celle-ci.

13. Molécule constituée d'un dérivé (guanidyl)-oligosaccharidique selon la revendication 12 pour utilisation en tant que support de macromolécules choisies parmi des protéines, oligonucléotides, ARNsi, gènes, agents thérapeutiques polymères, dendrimères.

14. Molécule constituée d'un dérivé (guanidyl)-oligosaccharidique selon la revendication 12 pour utilisation en tant que support de structures colloïdales supramoléculaires choisies parmi des charges supramoléculaires, liposomes, micelles, nanoparticules.

15. Procédé de préparation d'une molécule constituée d'un dérivé (guanidyl)-oligosaccharidique selon l'une des revendications 1 à 11 comprenant au moins les étapes suivantes :
- la dérivation des oligosaccharides sur le carbone anomère de l'unité glycosidique terminale avec un espaceur de formule -HN-R"'-Y, où R"' est une chaîne alkyle en C₈-C₂₂ linéaire ou ramifiée ou un dérivé de chaîne oligo(éthylène glycol) de formule-[CH₂CH₂O]ₘ-(CH₂)ₚ où m est un entier choisi dans la plage de 2 à 136 et p est un entier choisi dans la plage de 2 à 6, et Y est un groupe de terminaison choisi parmi les groupes carboxyle, amino, hydroxyle ou thiol ;
- la N-acétylation du dérivé oligoglycosylamino obtenu ;
- l'estérification des groupes hydroxyle de l'oligosaccharide avec un lieur de formule -CO-R'-X, où R' est une chaîne ramifiée alkyle en C₂-C₃ et X est un halogène ;
- la conjugaison d'un résidu guanidyle de formule -[CH₂C(CH₃)-CO-NH-R"-NHC(NH₂)=NH]-X, où R" est une chaîne alkyle en C₄-C₈ linéaire ou ramifiée portant éventuellement un groupe -OH ou un groupe -COOH, aux lieurs préalablement introduits sur les groupes hydroxyles du squelette oligosaccharidique via une polymérisation radicalaire par transfert d'atomes (ATRP).
